# EUROPEAN PATENT APPLICATION

(11) **EP 2 578 565 A1**
(43) Date of publication of application: **10.04.2013**
(21) Application number: 11786602.0
(22) Date of filing: 23.05.2011
(51) Int. Cl.: C07C 67/31, C07C 69/732, C07B 61/00

(54) **ALPHA-SUBSTITUTED ACRYLATE ESTERS, COMPOSITION CONTAINING SAME, AND METHOD FOR PRODUCING THE -SUBSTITUTED ACRYLATE ESTERS**

(30) Priority: 24.05.2010 JP 2010118759
(71) Applicant: Nippon Shokubai Co., Ltd., Osaka-shi, Osaka 541-0043 (JP)
(72) Inventor: KAWAMOTO Takahiro, Suita-shi Osaka 564-8512 (JP); TACHIBANA Atsushi, Suita-shi Osaka 564-8512 (JP)
(74) Representative: Hart-Davis, Jason
(86) International application number: PCT/JP2011/061776
(87) International publication number: WO 2011/148903

(57) **Abstract**

A method for producing α-substituted acrylate esters is provided which can be suitably used as an industrial method for producing α-substituted acrylate esters because the method does not have problems of apparatus corrosion or does not require waste detoxification and allows reaction in a short time with high yield. The method for producing an α-substituted acrylate ester includes a step of carrying out a reaction of a compound having a specific structure and an active hydrogen-containing compound under a condition where a tertiary amine and an acid and/or a salt thereof coexist.

## Description

### TECHNICAL FIELD

The present invention relates to a method for producing α-substituted acrylate esters and the α-substituted acrylate esters obtained thereby. More specifically, the present invention relates to a method for producing α-substituted acrylate esters useful for various applications such as optical materials, paint, reactive diluents, starting materials for surfactants, intermediates for production of pharmaceuticals/agrichemicals, starting materials for resists and the like and the α-substituted acrylate esters obtained thereby.

### BACKGROUND ART

α-substituted acrylate esters are a group of compounds which have been receiving attention because they are useful for various applications such as optical materials, paint, reactive diluents, starting materials for surfactants, intermediates for production of pharmaceuticals/agrichemicals, starting materials for resists and the like. Research has been actively carried out on such α-substituted acrylate esters and various synthesis methods thereof have been attempted before now.

Conventional production methods of α-substituted acrylate esters which have been disclosed are, for example, a method in which methyl α-(bromomethyl)acrylate and an alcohol are reacted in the presence of triethylamine to produce the corresponding methyl α-(alkoxymethyl)acrylate (see, for example, Non Patent Literature 1). Other disclosed methods include a method in which α-(hydroxymethyl)acrylate ester and a hydroxy group-containing compound are reacted in the presence of an acid catalyst to produce the corresponding α-(alkoxymethyl)acrylate ester (see, for example, Patent Literature 1), a method in which ethyl α-(hydroxymethyl)acrylate and ethanol are reacted in the presence of concentrated sulfuric acid to produce ethyl α-(ethoxymethyl)acrylate (see, for example, Non Patent Literature 2) and a method in which methyl α-(hydroxymethyl)acrylate and an alcohol are reacted in the presence of a solid acid, montmorillonite, to produce the corresponding ethyl α-(alkoxymethyl)acrylate (see, for example, Non Patent Document 3). Further, a method in which methyl acrylate and formaldehyde are reacted in the presence of triethylenediamine to produce the corresponding methyl α-(alkoxymethyl)acrylate (see, for example, Patent Literature 2) and a method in which methyl α-(hydroxymethyl)acrylate and an alcohol are reacted in the presence of a tertiary amine to produce the corresponding ethyl α-(alkoxymethyl)acrylate (see, for example, Patent Literature 3) have been disclosed.

### CITATION LIST

### - Patent Literature

Patent Literature 1: Japanese Patent Publication No. 3907738 (pages 1 to 2)
Patent Literature 2: U.S. Patent Specification No. 4,889,948
Patent Literature 3: Japanese Patent Publication No. 3943180 (pages 1 to 2)

### - Non Patent Literature

Non Patent Literature 1: Bunichiro Yamada and two others, "Makromolekulare Chemie", 1991, Vol. 192, p. 2713-2722
Non Patent Literature 2: M.G. Baldwin and one other, "Journal of Polymer Science: Part A", 1963, Vol. 1, p. 1919-1926
Non Patent Literature 3: Ponnusamy Shanmugam and one other, "Chemistry Letters", 2002, Vol. 31, p. 1212-1213

### SUMMARY OF INVENTION

### - Problems to be resolved by the Invention

Although various production methods for α-substituted acrylate esters are disclosed as described above, there is still room for improvements in terms of reaction rate, reaction yield and the like in order to obtain further effective reactions. The production method disclosed in Non Patent Literature 1 described above is the method in which highly reactive halomethyl acrylate esters are used as starting materials to obtain α-substituted acrylate esters in high yield. However, as halomethyl acrylate esters are expensive and difficult to obtain, this method has economical problems for industrial production. In addition, as high amount of hydrogen halides and ammonium salts thereof are generated as byproducts, thereby the method has problems such that corrosion of apparatus develops and that the waste needs to be detoxified.
In case of reaction between α-(hydroxymethyl)acrylate esters and a hydroxy group-containing compound in the presence of an acid catalyst as disclosed in Patent Literature 1 and Non Patent Literature 2, hydrolysis reaction of the α-(alkoxymethyl)acrylate ester products proceed while the α-(alkoxymethyl)acrylate ester products are produced. Therefore, the reaction has low yield and is far from effective. Further, because the side reaction, transesterification reaction, proceeds while α-(alkoxymethyl)acrylate esters are produced, selectivity of products is low when the structure of the ester substituent is different from the structure of the moiety other than the hydroxy group in the hydroxy group-containing compound. The method of Non Patent Literature 3 uses the solid acid, montmorillonite, as a catalyst and the solid acid is required in the high amount as 60% by mass of the reaction materials in this case. Therefore this method has problems as an industrial production method.
In case where methyl α-(hydroxymethyl)acrylate is used as a starting material with a tertiary amine as a catalyst as Patent Literatures 2 and 3, transesterification reaction proceeds together with the production reaction as above, in addition, long reaction time is required with low material conversion rate and product yield.
As described above, the methods conventionally proposed for production of α-substituted acrylate esters have room for improvement in order to make them more suitable for industrial production methods.

With the foregoing in view, it is an object of the present invention to provide a method for producing α-substituted acrylate esters which is suitable as an industrial method for producing α-substituted acrylate esters because it does not have problems of apparatus corrosion, does not require detoxification of wastes and allows reaction from inexpensive materials in a short time with high yield.

### - Means of solving the Problems

The present inventors have carried out various studies on methods for producing α-substituted acrylate esters and focused on the catalyst system used during reaction with compounds having specific structures as starting materials. As a result, they have found that, when a tertiary amine is used as a catalyst, co-existence of an acid allows reaction in a short time with high yield and the above problem can be clearly solved, thereby they achieve the present invention. Despite of the fact that it is predicted that co-existence of an acid in the system where a basic substance such as a tertiary amine is used as a catalyst may result in decrease in catalytic activity of the tertiary amine, the reaction actually proceeds more effectively under such configuration. This effect can be said to be unexpected and surprising and the technical significance of the present invention lies in this respect that the method having such an effect has been found out.

The present inventors have also found that α-substituted acrylate esters obtained by the method of the present invention and conventionally known production methods contain byproducts produced during production process and that α-substituted acrylate esters produced by the method of the present invention contain less byproducts than those produced by the conventionally known methods. Thus the α-substituted acrylate esters produced by the method of the present invention have superior process stability and storage stability, and are more suitably applied to various applications described above.

Thus the present invention is a method for producing an α-substituted acrylate ester represented by the following general formula (2):

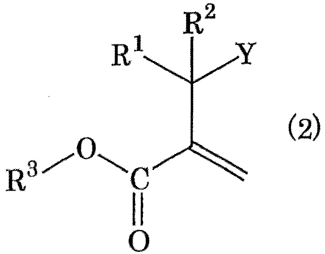

(wherein R¹, R² and R³ are the same or different and represent a hydrogen atom or an organic group having 1 to 200 carbon atoms; and Y represents a residue of an active hydrogen-containing compound), obtained by reacting a compound represented by the following general formula (1);

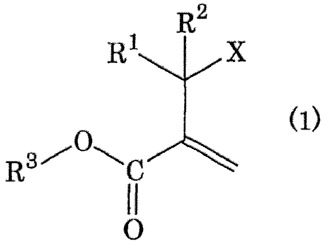

(wherein R¹, R² and R³ are the same as those in the above general formula (2); and X represents a Lewis base functional group) with the active hydrogen-containing compound,
wherein the method comprises a step of carrying out the reaction under a condition where a tertiary amine and an acid and/or a salt thereof coexist, and essentially comprises a reaction in which the Lewis base functional group in the compound represented by the general formula (1) is eliminated and replaced by the residue of the active hydrogen-containing compound.
The present invention is described in detail hereinbelow.

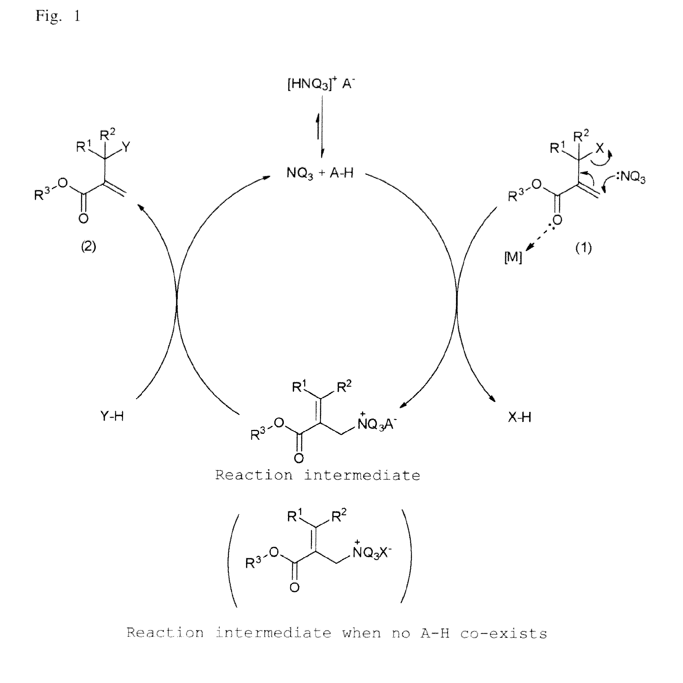

In the method for producing an α-substituted acrylate ester of the present invention, a reaction is essential in which the Lewis base functional group in the compound represented by the general formula (1) is eliminated and replaced by the residue of the active hydrogen-containing compound.
It is believed that the above reaction proceeds with the reaction mechanism shown in Fig. 1. Namely, the catalyst, tertiary amine (NQ₃) attacks the double bond moiety in the compound represented by the general formula (1) in a nucleophilic manner according to the S_{N}2' mechanism to generate a reaction intermediate which then reacts with the active hydrogen-containing compound (Y-H) to produce the compound represented by the general formula (2) . When a Lewis acid ([M]) co-exists therein as the acid and/or the salt thereof of the present invention, the double bond moiety of the compound represented by the general formula (1) increases electrophilicity, thereby promoting the reaction according to the above mechanism. When a Broensted acid (A-H) co-exists therein as the acid and/or the salt thereof of the present invention, a conjugate base (A⁻) of the Broensted acid works as a counter anion, the reaction is carried out by way of the reaction intermediate which is stable and has low basicity. Due to this, production of the reaction intermediate is promoted and side reactions are reduced which are otherwise promoted by bases such as transesterification reaction, hydrolysis reaction and the like. It is believed that, because the reaction proceeds according to such a presumed reaction mechanism, the reaction proceeds promptly and provides high yield and high selectivity even when low reactive substrates are used as starting materials. The present inventors have also found that inactivation reaction of the tertiary amine catalyst is suppressed when the acid and/or the salt thereof of the present invention co-exists, which may also explain that the high yield is obtained in a short time.
In Fig. 1, R¹, R² and R³ are the same or different and represent a hydrogen atom or an organic group having 1 to 200 carbon atoms. X represents the Lewis base functional group. X-H represents a byproduct which is a protonated Lewis base functional group X after elimination. NQ₃ represents the tertiary amine. Both A-H and [M] represent the acid and/or the salt thereof of the present invention; A-H represents a Broensted acid; A⁻ represents a conjugate base of the Broensted acid A-H; and [M] represents a Lewis acid. Y-H represents the active hydrogen-containing compound. Y represents a residue of the active hydrogen-containing compound.

The method for producing an α-substituted acrylate ester of the present invention comprises the step of reacting the compound represented by the following general formula (1);

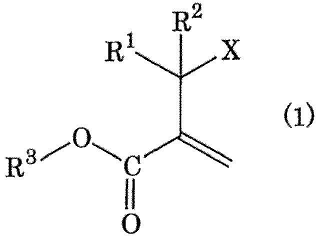

(wherein R¹, R² and R³ are the same or different and represent a hydrogen atom or an organic group having 1 to 200 carbon atoms; and X represents a Lewis base functional group) with the active hydrogen-containing compound under the condition where the tertiary amine and the acid and/or a salt thereof coexist. One or two or more of the compounds represented by the general formula (1), the active hydrogen-containing compounds, the tertiary amines and the acids and/or salts thereof may be respectively used. As long as the method of the present invention is carried out with the compound represented by the general formula (1), the active hydrogen-containing compound, the tertiary amine and the acid and/or a salt thereof, it may be carried out with other components, and as long as it comprises the step of reacting the compound represented by the general formula (1) with the active hydrogen-containing compound while the tertiary amine and the acid and/or a salt thereof coexist, it may comprise other steps.

In the method of the present invention, the compound represented by the general formula (1) and the active hydrogen-containing compound are reacted under the condition where the tertiary amine and the acid and/or a salt thereof coexist. It is sufficient that the tertiary amine and the acid and/or a salt thereof coexist at any time point during the reaction of the compound represented by the general formula (1) with the active hydrogen-containing compound; however, it is preferable that the reaction is carried out under the condition where the tertiary amine and the acid and/or a salt thereof coexist as long as possible and it is the most preferable that the tertiary amine and the acid and/or a salt thereof coexist from beginning to end of the reaction.

The tertiary amine and the acid and/or a salt thereof respectively may be added at one time at the beginning of the reaction or may be added sequentially with the progress of the reaction; it is preferable that they are added at one time at the beginning of the reaction. By adding them at one time at the beginning of the reaction, production of the intermediate can be promoted, the reaction can proceed promptly and the yield of the product can be increased.
When the tertiary amine and the acid and/or a salt thereof are added at one time at the beginning of the reaction, they may be added separately or may be mixed before addition.

In the compound represented by the general formula (1), X represents the Lewis base functional group, which may be a hydroxy, alkyloxy, alkenyloxy, aryloxy, acylarylamino, aroylarylamino, diacylamino, diaroylamino, acylaroylamino, alkylsulfonylamino, arylsulfonylamino, dialkylphosphonyl, dialkylphosphinyloxy, diarylphosphonyl, diarylphosphinyloxy or the like group. More preferably, it is a hydroxy, alkyloxy, alkenyloxy or aryloxy group and particularly preferably a hydroxy group. Namely, it is a suitable embodiment of the present invention that X in the compound represented by the general formula (1) is a hydroxy group.

The alkyloxy group may specifically include a methyloxy, ethyloxy, n- or i-propyloxy, n-, i- or t-butyloxy, n-, s- or t-amyloxy, neopentyloxy, n- or s-hexyloxy, n-heptyloxy, n-, s-or t-octyloxy, 2-ethylhexyloxy, capryloxy, nonyloxy, decyloxy, undecyloxy, lauryloxy, tridecyloxy, myristyloxy, pentadecyloxy, cetyloxy, heptadecyloxy, stearyloxy, nonadecyloxy, eicosyloxy, seryloxy, melissyloxy, cyclopentyloxy, cyclopentylmethyloxy, cyclohexyloxy, cyclohexylmethyloxy, 4-methylcyclohexyloxy, 4-t-butylcyclohexyloxy, tricyclodecanyloxy, isobornyloxy, adamantyloxy, dicyclopentanyloxy or the like group. Among these, methyloxy and ethyloxy groups are more preferred.

The alkenyloxy group may specifically include, a vinyloxy, allyloxy, methallyloxy, benzyloxy, dicyclopentenyloxy, crotyloxy, 1,1-dimethyl-2-propenyloxy, 2-methyl-butenyloxy, 3-methyl-2-butenyloxy, 3-methyl-3-butenyloxy, 2-methyl-3-butenyloxy, oleyloxy, linoleoxy, linolenoxy or the like group. Among these, vinyloxy, allyloxy, methallyloxy, benzyloxy and crotyloxy groups are more preferred.

The aryloxy group may specifically inlude a phenoxy, o-, m- or p-tolyloxy, o-, m- or p-methoxyphenyloxy, o-, m- or p-xylyloxy, cumenyloxy, mesityloxy, pentamethylphenyloxy, ethylphenyloxy, styryloxy, biphenyloxy, p-terphenyloxy, diphenylmethylphenyloxy, phenoxyphenyloxy, bibenzylyloxy, stilbenyloxy, indenyloxy, naphthyloxy, anthryloxy, phenanthryloxy or the like group. Among these, phenoxy, o-, m- or p-tolyloxy and o-, m- or p-methoxyphenyloxy groups are more preferred.

When the above X has a structure corresponding to a moiety of the general formula (1) other than X to which an oxygen atom binds, the compound represented by the general formula (1) has a form of an ether dimer represented by the following general formula (3);

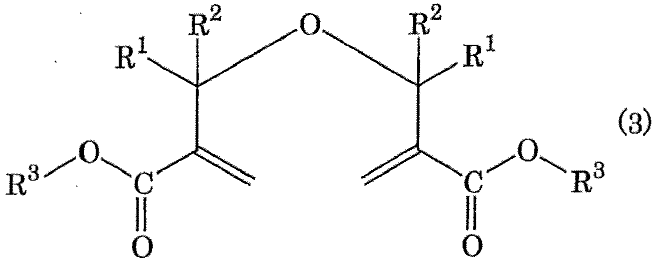

(wherein R¹, R² and R³ are the same as those in the general formula (1)). It is also a suitable embodiment of the present invention that X has the structure corresponding to a moiety of the general formula (1) other than X to which an oxygen atom binds.

In the compound represented by the general formula (1), R¹, R² and R³ are the same or different and represent a hydrogen atom or an organic group having 1 to 200 carbon atoms. The R¹, R² and R³ are not particularly limited and may be, for example, a hydrogen atom; a linear or branched alkyl group having 1 to 30 carbon atoms; a cycloalkyl group having 3 to 30 carbon atoms; a linear or branched alkenyl group having 2 to 30 carbon atoms; a cycloalkenyl group having 3 to 30 carbon atoms; a linear or branched alkynyl group having 2 to 30 carbon atoms; an aryl group having 6 to 30 carbon atoms; a polyalkylene glycol structure containing repetitive units of an oxyalkylene group having 4 to 200 carbon atoms; a hydroxy-substituted alkyl, alkenyl or aryl group having 1 to 30 carbon atoms; an alkyl, alkenyl or aryl group having 1 to 30 carbon atoms which is substituted by an alkoxy, alkenyloxy or aryloxy group having 1 to 30 carbon atoms; an alkyl, alkenyl or aryl group having 1 to 30 carbon atoms which is substituted by an alkylcarbonyloxy, alkenylcarbonyloxy or arylcarbonyloxy group having 1 to 30 carbon atoms; an alkyl, alkenyl or aryl group having 1 to 30 carbon atoms which is substituted by an alkyloxycarbonyl, alkenyloxycarbonyl or aryloxycarbonyl group having 1 to 30 carbon atoms; an alkyl, alkenyl or aryl group having 1 to 30 carbon atoms which is substituted by an alkylcarbonyl, alkenylcarbonyl or arylcarbonyl group having 1 to 30 carbon atoms; an alkyl, alkenyl or aryl group having 1 to 30 carbon atoms which is substituted by a cyano group; a carbonyl group substituted by an alkyl, alkenyl or aryl group having 1 to 30 carbon atoms; an oxycarbonyl group substituted by an alkyl, alkenyl or aryl group having 1 to 30 carbon atoms or the like. Among these, a hydrogen atom, a linear or branched alkyl group having 1 to 30 carbon atoms, a cycloalkyl group having 3 to 30 carbon atoms, a linear or branched alkenyl group having 2 to 30 carbon atoms and an aryl group having 6 to 30 carbon atoms are preferred.

The alkyl group may include a methyl, ethyl, n- or i-propyl, n-, i- or t-butyl, n-, s- or t-amyl, neopentyl, n-, s- or t-hexyl, n-, s- or t-heptyl, n-, s- or t-octyl, 2-ethylhexyl, capryl, nonyl, decyl, undecyl, lauryl, tridecyl, myristyl, pentadecyl, cetyl, heptadecyl, stearyl, nonadecyl, eicosyl, seryl, melissyl, cyclopropyl, cyclopentyl, cyclopentylmethyl, cyclohexyl, cyclohexylmethyl, 4-methylcyclohexyl, 4-t-butylcyclohexyl, tricyclodecanyl, isobornyl, adamantyl, dicyclopentanyl or the like group.
The alkenyl group may include a vinyl, allyl, metallyl, benzyl, crotyl, 1,1-dimethyl-2-propenyl, 2-methyl-butenyl, 3-methyl-2-butenyl, 3-methyl-3-butenyl, 2-methyl-3-butenyl, butenyl, pentenyl, hexenyl, heptenyl, octenyl, nonenyl, decenyl, undecenyl, dodecenyl, tridecenyl, tetradecenyl, pentadecenyl, hexadecenyl, heptadecenyl, oleyl, linoleyl, linolenyl, cyclopentenyl, cyclopentenylmethyl, cyclohexenyl, cyclohexenylmethyl, 4-methylcyclohexenyl, 4-t-butylcyclohexenyl, cyclooctenyl, dicyclopentenyl or the like group.
The aryl group may include a phenyl, o-, m- or p-tolyl, o-, m- or p-methoxyphenyl, o-, m- or p-xylyl, hemimellityl, cumenyl, pseudo-cumenyl, mesityl, junyl, pentaphenymethyl, ethylphenyl, cumenyl, styryl, biphenyl, p-terphenyl, diphenylmethylphenyl, phenoxyphenyl, bibenzylyl, stilbenyl, indenyl, naphthyl, anthryl, phenanthryl, furyl, thienyl or the like group.

Among these, the R¹ and R² are preferably a hydrogen atom or a methyl, ethyl, n-propyl, n-butyl, n-amyl, n-hexyl, n-heptyl, n-octyl, capryl, nonyl, decyl, undecyl, lauryl, tridecyl, myristyl, pentadecyl, cetyl, heptadecyl, stearyl, nonadecyl, eicosyl, seryl, melissyl, phenyl, tolyl, o-, m- or p-xylyl, biphenyl, terphenyl, phenoxyphenyl, bibenzylyl, indenyl, naphthyl or anthryl group. More preferably, they are a hydrogen atom or a methyl, ethyl, n-propyl, n-butyl or phenyl group and the most preferably R¹ and R² are both a hydrogen atom.

The R³ is preferably, among those mentioned above, a hydrogen atom or a methyl, ethyl, n- or i-propyl, n-, i- or t-butyl, n-, s- or t-amyl, neopentyl, n-, s- or t-hexyl, n-, s- or t-heptyl, n-, s- or t-octyl, 2-ethylhexyl, capryl, nonyl, decyl, undecyl, lauryl, tridecyl, myristyl, pentadecyl, cetyl, heptadecyl, stearyl, nonadecyl, eicosyl, seryl, melissyl, vinyl, allyl, metallyl, crotyl, 1,1-dimethyl-2-propenyl, 2-methylbutenyl, 3-methyl-2-butenyl, 3-methyl-3-butenyl, 2-methyl-3-butenyl, butenyl, pentenyl, hexenyl, heptenyl, octenyl, nonenyl, decenyl, undecenyl, dodecenyl, tridecenyl, tetradecenyl, pentadecenyl, hexadecenyl, heptadecenyl, oleyl, linoleic, linolenic, cyclopentyl, cyclopentylmethyl, cyclohexyl, cyclohexylmethyl, 4-methylcyclohexyl, 4-t-butylcyclohexyl, tricyclodecanyl, isobornyl, adamantyl, dicyclopentanyl, dicyclopentenyl, phenyl, methylphenyl, dimethylphenyl, trimethylphenyl, 4-t-butylphenyl, benzyl, diphenylmethyl, diphenylethyl, triphenylmethyl, cinnamyl, naphthyl, anthranyl, methoxyethyl, methoxyethoxyethyl, methoxyethoxyethoxyethyl, 3-methoxybutyl, ethoxyethyl, ethoxyethoxyethyl, cyclopentoxyethyl, cyclohexyloxyethyl, cyclopentoxyethoxyethyl, cyclohexyloxyethoxyethyl, dicyclopentenyloxyethyl, phenoxyethyl, phenoxyethoxyethyl, glycidyl, β-methylglycidyl, β-ethylglycidyl, 3,4-epoxycyclohexylmethyl, 2-oxycetanmethyl, 3-methyl-3-oxycetanmethyl, 3-ethyl-3-oxycetanmethyl, tetrahydrofuranyl, tetrahydrofurfuryl, tetrahydropyranyl, dioxazolanyl or dioxanyl group. It is more preferably a methyl, ethyl, n-butyl or 2-ethylhexyl group and the most preferably a methyl or ethyl group.

The active hydrogen-containing compound in the present invention is not particularly limited as long as it has an active hydrogen in its structure and may include alcoholic hydroxy group-containing compounds, phenolic hydroxy group-containing compounds, mercapto group-containing compounds, phosphinide group-containing compounds, carboxyl group-containing compounds, methylenebiscarbonyl group-containing compounds, nitro group-containing compounds, cyano group-containing compounds, methylenebiscyano group-containing compounds, azide group-containing compounds and the like. Namely, it is a suitable embodiment of the present invention that the active hydrogen-containing compound is at least one compound selected from the group consisting of alcoholic hydroxy group-containing compounds, phenolic hydroxy group-containing compounds, mercapto group-containing compounds, phosphinide group-containing compounds, carboxyl group-containing compounds, methylenebiscarbonyl group-containing compounds, nitro group-containing compounds, cyano group-containing compounds, methylenebiscyano group-containing compounds and azide group-containing compounds.
Further, it is a suitable embodiment of the present invention that an alcoholic hydroxy group-containing compound and/or a phenolic hydroxy group-containing compound is used.

The alcoholic hydroxy group-containing compounds can be represented by the following general formula (4);

**Y¹-OH** **(4)**

(wherein Y¹ represents an organic group having 1 to 200 carbon atoms). The same applies to Y¹ as the organic group having 1 to 200 carbon atoms of R¹, R² and R³ in the general formula (1).
The alcoholic hydroxy group-containing compounds represented by the general formula (4) may specifically include alkyl alcohols such as methanol, ethanol, propanol, n-butanol, t-butanol, 2-ethylhexanol and the like; cycloalkyl alcohols such as cyclopentanol, cyclohexanol and the like, aryl alcohols such as benzylalcohol and the like; unsaturated alcohols such as allyl alcohol, metallyl alcohol, crotyl alcohol, propargyl alcohol and the like; amino alcohols such as dimethylaminoethanol, diethylaminoethanol and the like; polymerizable group-containing alcohols such as 2-hydroxyethyl (meth)acrylate, 2-hydroxypropyl (meth)acrylate, 4-hydroxybutyl (meth)acrylate, methyl 2-(hydroxymethyl)acrylate, ethyl 2-(hydroxymethyl)acrylate and the like, alkylene glycol monoalkyl ethers such as methyl cellosolve, ethyl cellosolve, butyl cellosolve, carbitol, polyethylene glycol monomethoxy ether, polypropylene glycol monoethoxy ether and the like, alkylene glycols such as ethylene glycol, propylene glycol, diethylene glycol, polyethylene glycol, polypropylene glycol and the like, polyalcohols such as glycerin, trimethylolpropane, pentaerythritol and the like, saccharides such as glucose, mantose and the like, and halogen substitutes thereof. Among these, methanol, ethanol, propanol, n-butanol, 2-ethylhexanol, allyl alcohol, metallyl alcohol and crotyl alcohol are more preferred. Allyl alcohol, metallyl alcohol, and crotyl alcohol are particularly preferred.

The phenolic hydroxy group-containing compounds are the compounds represented by the above general formula (4) in which the hydroxy group is the substituent on a benzene ring.
The phenolic hydroxy group-containing compounds may specifically include phenol, o-, m- or p-cresol, o-, m- or p-methoxyphenol, 2,3-dimethylphenol, 2,4-dimethylphenol, 2,5-dimethylphenol, 2,6-dimethylphenol, 3,4-dimethylphenol, 3,5-dimethylphenol, trimethylphenol, o-, m- or p-t-butylphenol, o-, m- or p-s-butylphenol, methyl-t-butylphenol, o-, m- or p-cyclohexylphenol, o-, m- or p-phenylphenol, 1- or 2-naphthol, anthrol and the like. Among these, phenol, o-, m- or p-cresol, 2,3-dimethylphenol, 2,4-dimethylphenol, 2,5-dimethylphenol, 2,6-dimethylphenol, 3,4-dimethylphenol, 3,5-dimethylphenol, trimethylphenol, o-, m- or p-t-butylphenol, o-, m- or p-phenylphenol, 1- or 2-naphthol and anthrol are more preferred.

The mercapto group-containing compounds can be represented by the following general formula (5);

**Y²—SH** **(5)**

(wherein Y² represents an organic group having 1 to 200 carbon atoms). The same applies to Y² was the organic group having 1 to 200 carbon atoms of R¹, R² and R³ in the general formula (1).
The mercapto group-containing compounds represented by the above general formula (5) may specifically include methyl mercaptan, ethyl mercaptan, n- or i-propyl mercaptan, n-, i- or t-butyl mercaptan, thiophenol and the like. Among these, methyl mercaptan, ethyl mercaptan, n- or i-propyl mercaptan, n-, i- or t-butyl mercaptan and thiophenol are more preferred.

The phosphinide group-containing compounds can be represented by the following general formula (6);

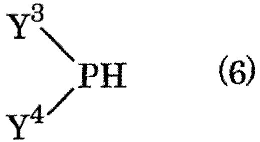

(wherein Y³ and Y⁴ are the same or different and represent an organic group having 1 to 200 carbon atoms). The same applies to Y³ and Y⁴ as the organic group having 1 to 200 carbon atoms of R¹, R² and R³ in the general formula (1).
The phosphinide group-containing compounds represented by the above general formula (6) may include diaryl phosphines, alkylaryl phosphines, dialkyl phosphines, dicycloalkyl phosphines, arylcycloalkyl phosphines, alkylcycloalkyl phosphines and the like. They may specifically inlcude diphenyl phosphine, di(3-methylphenyl)phosphine, di(4-methylphenyl)phosphine, bis(2,4-dimethylphenyl)phosphine, bis(3,5-dimethylphenyl)phosphine, bis(2,6-dimethylphenyl)phosphine, bis(2,4,6-trimethylphenyl)phosphine, bis(3,4,5-trimethylphenyl)phosphine, di(4-t-butylphenyl)phosphine, bis(3,5-di-t-butylphenyl)phosphine, di(3-methoxyphenyl)phosphine, di(4-methoxyphenyl)phosphine, bis(2,4-dimethoxyphenyl)phosphine, bis(3,5-dimethoxyphenyl)phosphine, bis(2,6-dimethoxyphenyl)phosphine, bis(2,4,6-trimethoxyphenyl)phosphine, bis(3,4,5-trimethoxyphenyl)phosphine, bis(3,5-dimethyl-4-methoxyphenyl)phosphine, di(3-chlorophenyl)phosphine, di(4-chlorophenyl)phosphine, bis(2,4-dichlorophenyl)phosphine, bis(3,5-dichlorophenyl)phosphine, bis(2,6-dichlorophenyl)phosphine, bis(2,4,6-trichlorophenyl)phosphine, bls(3,4,5-trichlorophenyl)phosphine, di(3-fluorophenyl)phosphine, di(4-fluorophenyl)phosphine, bis(2,4-difluorophenyl)phosphine, bis(3,5-difluorophenyl)phosphine, bis(2,6-difluorophenyl)phosphine, bis(2,4,6-trifluorophenyl)phosphine, bis(3,4,5-trifluorophenyl)phosphine, di(3-trifluoromethylphenyl)phosphine, di(4-trifluoromethylphenyl)phosphine, bis(2,4-bistrifluoromethylphenyl)phosphine, bis(3,5-bistrifluoromethylphenyl)phosphine, bis(2,6-bistrifluoromethyl-phenyl)phosphine, bis(2,4,6-tristrifluoromethylphenyl)phosphine, bis(3,4,5-tristrifluoromethylphenyl)phosphine, di(3-dimethylaminophenyl)phosphine, di(4-dimethylaminophenyl)phosphine, bis(2,4-bisdimethylaminophenyl)phosphine, bis(3,5-bisdimethylaminophenyl)phosphine, bis(2,6-bisdimethylaminophenyl)phosphine, bis(2,4,6-trisdimethylaminophenyl)phosphine, bis(3,4,5-trisdimethylaminophenyl)phosphine, di(3-biphenyl)phosphine, di(4-biphenyl)phosphine, bis(2,4-diphenylphenyl)phosphine, bis(3,5-diphenylphenyl)phosphine, bis(2,6-diphenylphenyl)phosphine, bis(2,4,6-triphenylphenyl)phosphine, bis(3,4,5-triphenylphenyl)phosphine, di(α-naphthyl)phosphine, di(β-naphthyl)phosphine, di(6-methoxy-α-naphthyl)phosphine, di(6-methoxy-p-naphthyl)phosphine, di(3,4-methylenedioxyphenyl)phosphine, dimethyl phosphine, diethyl phosphine, diisopropyl phosphine, di-t-butyl phosphine, dicyclopropyl phosphine, dicyclopentyl phosphine, dicyclohexyl phosphine, dicyclooctyl phosphine, dicyclodecyl phosphine, dicyclododecyl phosphine, cyclohexylphenyl phosphine and the like. Among these, diphenyl phosphine, dimethyl phosphine, diethyl phosphine, diisopropyl phosphine, di-t-butyl phosphine, dicyclopropyl phosphine, dicyclopentyl phosphine, dicyclohexyl phosphine, dicyclooctyl phosphine, dicyclodecyl phosphine and di-cyclododecyl phosphine are more preferred.

The carboxyl group-containing compounds can be represented by the following general formula (7);

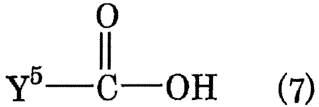

(wherein Y⁵ represents an organic group having 1 to 200 carbon atoms). The same applies to Y⁵ as the organic group having 1 to 200 carbon atoms of R¹, R² and R³ in the general formula (1).
The carboxyl group-containing compounds represented by the above general formula (7) may specifically include formic acid, acetic acid, propionic acid, butyric acid, isobutyric acid, oxalic acid, malonic acid, succinic acid, glutaric acid, maleic acid, fumaric acid, benzoic acid, phthalic acid, 1,2,3-benzenetricarboxylic acid, glycolic acid, lactic acid, malic acid, citric acid, salicylic acid, (meth) acrylic acid and the like. Among these, formic acid, acetic acid, butyric acid, oxalic acid, maleic acid, fumaric acid, benzoic acid, phthalic acid, acrylic acid and methacrylic acid are more preferred.

The methylenebiscarbonyl group-containing compounds can be represented by the following general formula (8);

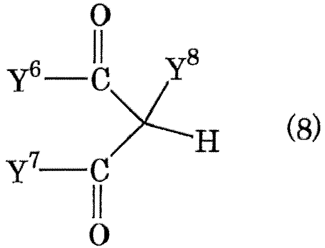

(wherein Y⁶, Y⁷ and Y⁸ are the same or different and represent an organic group having 1 to 200 carbon atoms). The same applies to Y⁶, and Y⁸ as the organic group having 1 to 200 carbon atoms of R¹, R² and R³ in the general formula (1).
The methylenebiscarbonyl group-containing compounds represented by the above general formula (8) may specifically include 2,4-pentadione, 3,5-heptadione, 1,3-diphenylpropane dione, dimethyl malonate, diethyl malonate, dipropyl malonate, diisopropyl malonate, dibutyl malonate, 2-methyl malonate and the like. Among these, 2,4-pentadione, 3,5-heptadione, 1,3-diphenylpropane dione, dimethyl malonate, diethyl malonate, dimethyl 2-methylmalonate, diethyl 2-methylmalonate are more preferred.

The nitro group-containing compounds can be represented by the following general formula (9);

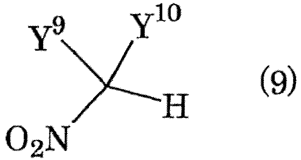

(wherein Y⁹ and Y¹⁰ are the same or different and represent an organic group having 1 to 200 carbon atoms). The same applies to Y⁹ and Y¹⁰ as the organic group having 1 to 200 carbon atoms of R¹, R² and R³ in the general formula (1).
The nitro group-containing compounds represented by the above general formula (9) may specifically include nitromethane, nitroethane, nitropropane, methyl nitroacetate, ethyl nitroacetate, propyl nitroacetate, butyl nitroacetate, nitromethyl phenyl sulfone and the like. Among these, nitromethane, nitroethane and nitropropane are more preferred.

The cyano group-containing compounds may include hydrogen cyanide and the compounds represented by the following general formula (10);

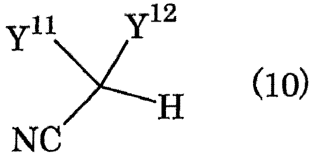

(wherein Y¹¹ and Y¹² are the same or different and represent an organic group having 1 to 200 carbon atoms). The same applies to Y¹¹ and Y¹² as the organic group having 1 to 200 carbon atoms of R¹, R² and R³ in the general formula (1).
The cyano group-containing compounds represented by the above general formula (10) may specifically include cyanomethane, cyanoethane, methyl cyanoacetate, ethyl cyanoacetate, propyl cyanoacetate, butyl cyanoacetate and the like.
Among the above cyano group-containing compounds, hydrogen cyanide, methyl cyanoacetate and ethyl cyanoacetate are more preferred.

The methylenebiscyano group-containing compounds can be represented by the following general formula (11);

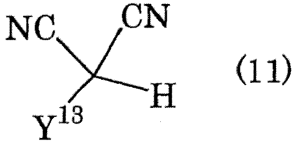

(wherein Y¹³ represents an organic group having 1 to 200 carbon atoms). The same applies to Y¹³ as the organic group having 1 to 200 carbon atoms of R¹, R² and R³ in the general formula (1).
The methylenebiscyano group-containing compounds represented by the above general formula (11) may specifically include malononitrile, 2-methylmalononitrile, 2-ethylmalononitrile and the like. Among these, malononitrile and 2-methylmalononitrile are more preferred.
The azide group-containing compounds may include hydrogen azide.

The reaction in which the alcoholic hydroxy group-containing compound or phenolic hydroxy group-containing compound represented by the above general formula (4) and/or the mercapto group-containing compound represented by the above general formula (5) is used as the active hydrogen-containing compound with the catalyst which is an acid as described in Patent Literature 1 and Non Patent Literature 2 allows side reactions such as transesterification reaction and thioesterification reaction. Because of this, when the ester substituent of the compound represented by the general formula (1), i.e., R³ in the general formula (1) is different from Y¹ in the general formula (4) and/or Y² in the general formula (5), selectivity of the reaction product may be reduced. In addition, because of the development of hydrolysis during the production reaction, the yield of the reaction may also be reduced. However, when the tertiary amine and the acid and/or the salt thereof coexist as catalysts in the reaction according to the method of the present invention, as mentioned above, side reactions and hydrolysis reactions are sufficiently suppressed even when the alcoholic hydroxy group-containing compound, phenolic hydroxy group-containing compound and/or mercapto group-containing compound are used as the active hydrogen-containing compound. The reason for this is presumed that the reaction may proceed with the mechanism as shown in Fig. 1.
Therefore, when the reaction materials used are the compound represented by the general formula (1) and the alcoholic hydroxy group-containing compound, phenolic hydroxy group-containing compound and/or mercapto group-containing compound, the superiority of the method of the present invention is more profoundly exhibited in case when the structure of the ester substituent moiety in the compound represented by the general formula (1) is different from the moiety other than the hydroxy group in the hydroxy group-containing compounds and/or the moiety other than the mercapto group in the mercapto group-containing compound. Namely, particularly when the active hydrogen-containing compound used is the alcoholic hydroxy group-containing compound and/or phenolic hydroxy group-containing compound represented by Y¹-OH (wherein Y¹ represents an organic group having 1 to 200 carbon atoms), it is a suitable embodiment of the present invention that it is the compound wherein Y¹ is different from R³.

When the active hydrogen-containing compound used is the alcoholic hydroxy group-containing compound represented by the general formula (4) wherein the structure of Y¹ is the same as the moiety other than X in the general formula (1), an ether dimer of an α-substituted acrylate ester can be synthesized. Accordingly, the method of the present invention can also be suitably used for the synthesis of an ether dimer of an α-substituted acrylate ester.

The tertiary amine in the present invention may include tertiary amine compounds represented by the following general formula (12);

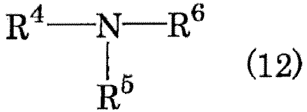

(wherein R⁴, R⁵ and R⁶ are the same or different and are a linear or branched alkyl group having 1 to 12 carbon atoms or a cycloalkyl group having 5 to 8 carbon atoms) ; tertiary amine compounds represented by the following general formula (13);

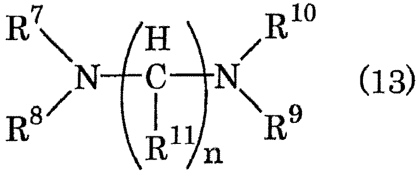

(wherein R⁷, R⁸, R⁹ and R¹⁰ are the same or different and are a linear or branched alkyl group having 1 to 12 carbon atoms or a cycloalkyl group having 5 to 8 carbon atoms; R¹¹ is a hydrogen atom or a methyl group; and n is an integer of 1 to 8); cyclic tertiary amine compounds having 3 to 15 carbon atoms; ion exchange resins having tertiary amine in the exchange group; polymers having tertiary amine and the like.

The tertiary amine compounds represented by the above general formula (12) may specifically include trimethylamine, triethylamine, tri-n-propylamine, tri-n-butylamine, tri-isobutylamine, N,N-diethylmethylamine, N,N-dimethylethylamine, N,N-dimethylpropylamine, N,N-dimethylisopropylamine, N,N-dimethylbutylamine, N,N-dimethylcyclohexylamine, N,N-dimethyl(2-ethylhexyl)amine, N,N-dimethyllaurylamine and the like.

The tertiary amine compounds represented by the above general formula (13) may specifically include N,N,N',N'-tetramethyldiaminomethane, N,N,N',N'-tetramethylethylenediamine, N,N,N',N'-tetramethyl-1,3-diaminopropane, N,N,N',N'-tetramethyl-1,3-diaminobutane, N,N,N',N'-tetramethyl-1,4-diaminobutane, N,N,N',N'-tetramethyl-1,6-hexanediamine and the like.

The cyclic tertiary amine compounds may specifically include 1-azabicyclo[2,2,1]heptane, 1-azabicyclo[3,2,1]octane, 1-azabicyclo[3,3,1]nonane, 1-azabicyclo[2,3,2]nonane, 1-azabicyclo[3,3,0]octane, 1-azabicyclo[4,3,0]nonane, quinuclidine, pyrrolidine, pyrocollidine, lupinane, quinuclidinone, 3-hydroxyquinuclidine, quinolidine, N-methylpyrrolidine, N-methylpyrroline, N-methylpiperidine, N,N'-dimethylpiperadine, N-(2-dimethylaminoethyl)-N'-methylpiperadine, triethylenediamine , hexamethylenediamine, N-methylimidazole, anisidine, toluidine, pyridine, 2,6-dimethylpyridine, 3,5-dimethylpyridine, N,N-dimethyl-4-pyridine, 1,8-diazabicyclo[5,4,0]undeca-7-ene, 1,5-diazabicyclo[4,3,0]nona-5-ene and the like.

The ion exchange resins having tertiary amine as the exchange group may specifically include Amberlite A-21, Amberlite IRA-68, Amberlite IRA-93ZU, Amberlinte IRA-35 and Amberlite IRA-99 from Rohm and Haas; Diaion WA-10, Diaion WA-11 and Diaion WA-30 from Mitsubishi Chemical Corp.; Dowex MWA-1, Dowex 66 and Dowex D-3 from Dow Chemical; Duolite A-368, Duolite A-561, Duolite A-340, Duolite A-375 and Duolite-378 from Sumitomo Chemical Co., Ltd. and the like.

The polymers having tertiary amine may specifically include polymers obtained by polymerizing a polymerizable monomer having a tertiary amine group such as N,N-dimethylaminoethyl (meth)acrylate, N,N-dimethylaminoethyl (meth)acrylamide and the like and may be homopolymers or copolymers with a polymerizable monomers such as alkyl (meth)acrylate, alkyl (meth)acrylamide, styrene or crosslinked products of these polymers and the like.

Among the above tertiary amines, the ones which is not bulky at the nitrogen atom are preferable such as trimethylamine, N,N-dimethylethylamine, N,N-dimethylpropylamine, N,N-dimethylbutylamine, N,N,N',N'-tetramethylethylenediamine, N,N,N',N'-tetramethyl-1,3-diaminopropane, N,N,N',N'-tetramethyl-1,3-diaminobutane, triethylenediamine, quinuclidine, pyrocollidine, N-methylimidazole, N,N-dimethyl-4-pyridine, 1,8-diazabicyclo[5,4,0]undeca-7-ene, 1,5-diazabicyclo[4,3,0]nona-5-ene and the like. Trimethylamine, triethylenediamine, quinuclidine and pyrocollidine are more preferred and triethylenediamine, quinuclidine and pyrocollidine are still more preferred.

The acid and/or the salt thereof in the present invention is not particularly limited as long as it has the properties as a Broensted acid or a Lewis acid, acts as an acid as a whole in a reaction solution, promotes the reaction in which a Lewis base functional group is eliminated from the compound represented by the general formula (1) and replaced with a residue of the active hydrogen-containing compound and does not inhibit nucleophilicity of the coexisting tertiary amine by forming a salt therewith, and may include organic acids such as formic acid, acetic acid, propionic acid, butyric acid, oxalic acid, benzoic acid, terephthalic acid, phthalic acid, maleic acids phenols, pentafluorophenol, trichlorophenols (TCPs), dichlorophenol, paramethoxyphenol, p-toluenesulfinic acid, p-toluenesulfonic acid, methanesulfonic acid and the like; inorganic acids such as hydrochloric acid, sulfuric acid, nitric acid, phosphoric acid, boric acid, vanadic acid, tungstic acid, molybdic acid and the like; salts or mixtures of the organic acids or inorganic acids with amines; boron compounds such as borate esters, boronic acids, boronic esters, boron halides, triarylborane and the like; Lewis acid-type metal salts such as zinc acetate, zinc triflate, zinc acetylacetonate, magnesium acetate, lanthanum acetate, lanthanum triflate, aluminum acetate, aluminum triflate, iron acetate, iron triflate and the like; heteropolyacids such as phosphotungstic acid, molybdophosphoric acid, tungstosilicic acid, molybdosilicic acid and the like; Broensted acids such as acidic ion exchange resins and the like. The amines in the salts or mixtures of the organic acids or inorganic acids with amines may be the same compounds as the tertiary amine described above.
Among these, organic acids having pKa of 3 to 8 such as formic acid, acetic acid, propionic acid, butyric acid, benzoic acid, terephthalic acid, phthalic acid, maleic acid, pentafluorophenol, trichlorophenols, dichlorophenol, p-toluenesulfinic acid and the like; inorganic acids such as boric acid, phosphoric acid and the like; salts or mixtures of organic acids such as formic acid, acetic acid, propionic acid, butyric acid, benzoic acid, terephthalic acid, phthalic acid, maleic acid, pentafluorophenol, trichlorophenols, dichlorophenol, oxalic acid, p-toluenesulfonic acid and the like or inorganic acids such as hydrochloric acid, boric acid, phosphoric acid, sulfuric acid, nitric acid, vanadic acid, tungstic acid, molybdic acid and the like with amines such as trimethylamine, triethylamine, N-methylimidazole, anisidine, toluidine, pyridine, 2,6-dimethylpyridine, 3,5-dimethylpyridine, N,N-dimethyl-4-pyridine, triethylenediamine, quinuclidine, pyrocollidine; boron compounds such as borate esters, boronic acids, boronic esters, boron halides, triarylborane and the like; Lewis acid-type metal salts such as zinc acetate, zinc triflate, zinc acetylacetonate, magnesium acetate, lanthanum acetate, lanthanum triflate, aluminum acetate, aluminum triflate and the like are preferred. Acetic acid, benzoic acid, pentafluorophenol, trichlorophenols, boric acid, borate esters, triarylboranes, zinc acetate, zinc triflate, salts or mixtures of these acids with trimethylamine, triethylamine, anisidine, toluidine, pyridine, triethylenediamine are more preferred and acetic acid, benzoic acid, trichlorophenols, boric acid, zinc acetate and zinc triflate are still more preferred. Acetic acid and zinc acetate are particularly preferred.

The mixing ratio between the compound represented by the general formula (1) and the active hydrogen-containing compound in the reaction step of the present invention can be appropriately selected according to the combination of the types of the compound represented by the general formula (1) and the active hydrogen-containing compound. However, the molar ratio between the compound represented by the general formula (1) and the active hydrogen-containing compound is preferably 10/1 to 1/50. When the mixing ratio is within this range, the reaction may proceed smoothly. It is more preferably 5/1 to 1/10 and still more preferably 2/1 to 1/5.

The amount of the tertiary amine to be used in the reaction step of the present invention is preferably, relative to 100 mol% of the compound represented by the general formula (1), 0.1 to 50 mol%. When the amount of the tertiary amine is less than 0.1 mol%, sufficient catalyst activity may not be obtained and when it is more than 50 mol%, side reactions may proceed. It is more preferably 0.5 to 20 mol% and still more preferably 1 to 10 mol%.

The amount of the acid and/or the salt thereof to be used in the reaction step of the present invention is preferably, relative to 100 mol% of the compound represented by the general formula (1), 0. 01 to 50 mol%. When the amount of the acid and/or the salt thereof is within this range, it is expected that the reaction promoting action is sufficiently demonstrated. It is more preferably 0.1 to 20 mol% and still more preferably 0.1 to 10 mol%.

The mixing ratio of the tertiary amine and the acid and/or the salt thereof in the reaction step of the present invention can be appropriately selected according to the combination of the types of the tertiary amine and the acid and/or the salt thereof. The molar ratio between the tertiary amine and the acid and/or the salt thereof can be in the range of 10000/1 to 1/10000. Among others, the molar ratio between the tertiary amine and the acid and/or the salt thereof is preferably in the range of 1000/1 to 1/100, more preferably 100/1 to 1/10 and still more preferably 20/1 to 1/5.
When the acid and/or the salt thereof used is a Broensted acid having pKa of less than 3 such as oxalic acid, p-toluenesulfonic acid, hydrochloric acid, phosphoric acid, sulfuric acid, nitric acid, vanadic acid, tungstic acid, molybdic acid or the like, the molar ratio between the tertiary amine and the acid and/or the salt thereof is particularly preferably 20/1 to 1/1. When the acid and/or the salt thereof used is a Broensted acid having pKa of 3 to 8 such as formic acid, acetic acid, propionic acid, butyric acid, benzoic acid, terephthalic acid, phthalic acid, maleic acid, pentafluorophenol, trichlorophenols, dichlorophenol or the like, the molar ratio between the tertiary amine and the acid and/or the salt thereof is particularly preferably 10/1 to 1/5. When the acid and/or the salt thereof used is a Broensted acid having pKa of more than 8 such as boric acid, paramethoxyphenol or the like, the molar ratio between the tertiary amine and the acid and/or the salt thereof is particularly preferably 10/1 to 1/10. When the acid and/or a salt thereof is a salt or mixture of the above organic acid or inorganic acid with the amines, the molar ratio between the tertiary amine and the acid and/or the salt thereof is particularly preferably 5/1 to 1/10. When the acid and/or the salt thereof used is a boron compound such as borate esters, triarylboranes or a Lewis acid such as Lewis acid-type metal salts, e.g., zinc acetate, zinc triflate, the molar ratio between the tertiary amine and the acid and/or the salt thereof is particularly preferably 20/1 to 112.

The reaction conditions in the above reaction step are not particularly limited. However, because the reaction material, compound represented by the general formula (1), and the product, α-substituted acrylate ester represented by the general formula (2), are both highly polymerizable, the reaction step is preferably carried out with addition of a polymerization inhibitor and/or a molecular oxygen in the reaction system in order to prevent the development of the polymerization reaction during the reaction step.

The polymerization inhibitor may include, for example, hydroquinone, hydroquinone monomethyl ether, p-benzoquinone, t-butylcatechol, t-butylhydroquinone, 2,6-di(t-butyl)-4-methylphenol, phenothiazine, 2,2,6,6-tetramethylpiperidine-1-oxyl, 4-hydroxy-2,2,6,6-tetramethylpiperidine-1-oxyl and the like. One or two or more of these polymerization inhibitors may be used. The amount of the polymerization inhibitor can be appropriately selected without particular limitation and is preferably, relative to 100% by mass of the compound represented by the general formula (1), 0.001 to 10% by mass and more preferably 0.01 to 5% by mass.

The molecular oxygen to be used may be, for example, molecular oxygen; mixed gas of molecular oxygen with inert gas such as nitrogen, argon and the like; and air. The molecular oxygen can be introduced by dissolving or blowing (so-called bubbling) thereof in the reaction system.
The amount of the molecular oxygen to be fed is not particularly limited. However, it is preferably fed such that the oxygen concentration in the gas phase of the reaction system is 0.01 to 10% by volume.
In order to sufficiently suppress the polymerization reaction in the reaction step described above, it is preferable that the polymerization inhibitor and the molecular oxygen are used in combination.

The reaction step may be carried out in a solvent. The solvent is not particularly limited as long as it does not inhibit the reaction and may include, for example, ketones such as acetone, methyl ethyl ketone, methyl propyl ketone, methyl isobutyl ketone, methyl amyl ketone, methyl cyclohexyl, ketone, diethyl ketone, ethyl butyl ketone, trimethyl nonanone, acetonitrileacetone, dimethyloxide, phorone, cyclohexanone and the like; ethers such as diethyl ether, dipropyl ether, diisopropyl ether, dibutyl ether, diamyl ether, diethyl acetal, dihexyl ether, t-butyl methyl ether, cyclopentyl methyl ether, tetrahydrofuran, tetrahydropyran, trioxane, dioxane and the like; esters such as methyl acetate, ethyl acetate, propyl acetate, butyl acetate, i-propyl acetate, t-butyl acetate and the like; aromatic hydrocarbons such as benzene, toluene, xylene, chlorobenzene and the like; aliphatic hydrocarbons such as cyclohexane, hexane, methylcyclohexane, heptane, octane and the like; water and the like. Among these, it is preferable to use dipropyl ether, diisopropyl ether, dibutyl ether, tetrahydrofuran, tetrahydropyran, dioxane, toluene, xylene, cyclohexane, hexane, methylcyclohexane, heptane and octane. One or two or more of these solvents may be used.

The amount of the solvent to be used is not particularly limited and is preferably, relative to 100% by mass of the compound represented by the general formula (1), 10000 to 0% by mass, more preferably 1000 to 0% by mass and still more preferably 100 to 0% by mass.

Reaction temperature in the above reaction step is preferably 0 to 150°C. When the reaction temperature is lower than 0°C, reaction time becomes too long to carry out the reaction effectively. On the other hand, when the reaction temperature is higher than 150°C, the polymerization reaction described above may not be sufficiently suppressed. The reaction temperature is more preferably 50 to 120°C. Reaction time may be appropriately selected according to the reaction temperature, the type, combinations and the amount used of the compound represented by the general formula (1), the active hydrogen-containing compound, the tertiary amine, the acid and/or the salt thereof and the solvent so that the reaction in the reaction step is completed. Reaction pressure is not particularly limited and may be any condition of under normal pressure (atmospheric pressure), reduced pressure or increased pressure.

In the reaction step, it is preferable to remove (distill off) the byproduct (X-H in Fig. 1) which is the eliminated and then protonated Lewis base functional group of the compound represented by the general formula (1) from the reaction system. Because the reaction proceeding in the reaction step is an equilibrium reaction, removal of the byproduct (X-H in Fig. 1) from the reaction system can shift the equilibrium of the reaction toward the production system and promote the reaction, thereby further increasing the reaction yield and the material conversion rate.
Particularly when the Lewis base functional group in the compound represented by the general formula (1) is a hydroxy group, the above reaction step corresponds to dehydraion reaction. Therefore, it is preferable to promptly remove water produced by the reaction from the reaction system.
The method for removing the byproduct from the reaction system is not particularly limited and may include, for example, distillation at normal or reduced pressure, azeotropic removal with the active hydrogen-containing compound and/or the solvent, removal by membrane separation, removal by adsorption, removal by extraction, removal by filtration and the like. Among these, azeotropic removal with the active hydrogen-containing compound and/or the solvent is preferable.

In the azeotropic removal with the active hydrogen-containing compound and/or the solvent, the active hydrogen-containing compound and/or the solvent act as, in addition to their own functions, azeotropic agents. Alternatively, in addition to the compounds added to the reaction system as the active hydrogen-containing compound and the solvent, a compound exemplified as the active hydrogen-containing compound or the solvent may be further added as an azeotropic agent in order to remove the byproduct. However, because separate addition of an azeotropic agent is not required if the active hydrogen-containing compound and/or the solvent act as azeotropic agents, the byproduct is preferably distilled off by azeotropic distillation with the active hydrogen-containing compound and/or the solvent.
The azeotropic agent forms a minimum azeotrope with a component to be distilled off (byproduct), thereby allowing separation between two components (the byproduct and the active hydrogen-containing compound and the like) which cannot be separated otherwise in a normal distillation.

The azeotropic agent may be the compounds exemplified above as the active hydrogen-containing compound or the solvent and can be appropriately selected according to the type of the reaction material, the active hydrogen-containing compound, the solvent and the like. Among these, diisopropyl ether, dipropyl ether, dibutyl ether, tetrahydrofuran, tetrahydropyran, dioxane, toluene, xylene, cyclohexane, hexane, methylcyclohexane, heptane and octane are preferably used. More preferably, it is diisopropyl ether, toluene and cyclohexane. When the active hydrogen-containing compound used is allylalcohol, the azeotropic agent is preferably diisopropyl ether.
When the azeotropic agent is further added in addition to the compounds added as the active hydrogen-containing compound and the solvent to the reaction system as described above in order to remove the byproduct, the amount of the azeotropic agent added is preferably, relative to 100% by mass of the active hydrogen-containing compound, 1 to 100% by mass. It is more preferably 10 to 50% by mass.

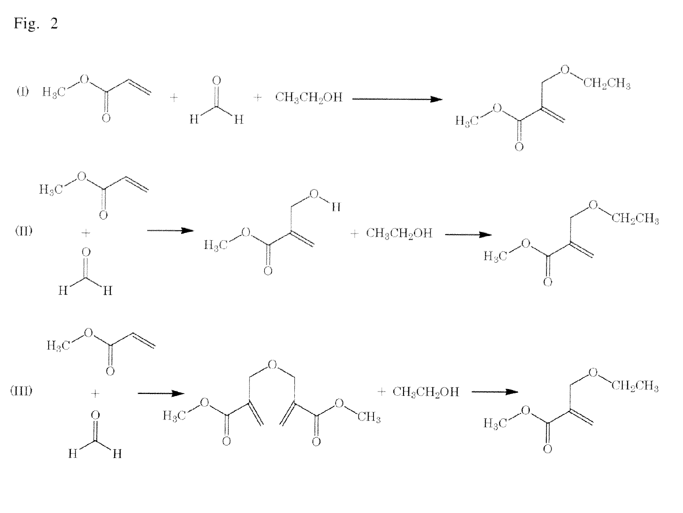

As described hereinbelow, the α-substituted acrylate ester represented by the general formula (2) may be synthesized by charging starting materials for synthesis of the compound represented by the general formula (1) and the active hydrogen-containing compound, the tertiary amine and the acid and/or the salt thereof to be used in the present invention at one time into the reaction system or by synthesizing the compound represented by the general formula (1) and then adding, without purification, the active hydrogen-containing compound, the tertiary amine and the acid and/or the salt thereof to be used in the present invention to the reaction system. In such syntheses of the α-substituted acrylate ester, the compound represented by the general formula (1) is produced as the intermediate. Therefore, the direct synthesis of the α-substituted acrylate ester represented by the general formula (2) without removing the compound represented by the general formula (1) between the synthesis of the compound represented by the general formula (1) and the synthesis of the α-substituted acrylate ester represented by the general formula (2) is also within the scope of the present invention. Such a reaction which is schematically represented by exemplifying the case in which methyl acrylate and formaldehyde are used as the starting materials for synthesis of the compound represented by the general formula (1) and ethanol is used as the active hydrogen-containing compound may include the reaction pathway shown in Fig. 2, for example. The reaction formula (I) shows the one-step synthesis from the acrylate ester and the aldehyde and the reaction formula (II) shows the synthetic pathway in which methyl hydroxymethylacrylate is synthesized as the intermediate. The reaction formula (III) shows the synthetic pathway in which an ether dimer of methyl hydroxymethylacrylate is synthesized as the intermediate.

The α-substituted acrylate ester obtained by the method of the present invention is represented by the following formula (2);

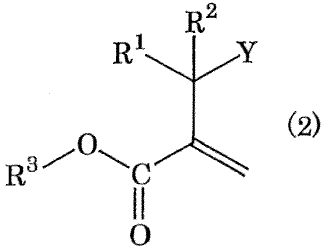

(wherein R¹, R² and R³ are the same as those in the above general formula (1); and Y represents a residue of the active hydrogen-containing compound).
In this formula, Y represents a residue of the active hydrogen-containing compound, which can be specifically represented as follows.
When the active hydrogen-containing compound is the alcoholic hydroxy group-containing compound or phenolic hydroxy group-containing compound, it is represented by the following general formula (14);

**Y¹-O-_{c}** **(14)**

(wherein Y¹ is the same as Y¹ in the general formula (4); and c corresponds to the carbon atom to which R¹, R² and Y bind in the general formula (2)). When the active hydrogen-containing compound is the mercapto group-containing compound, it is represented by the following general formula (15);

**Y²-S-_{c}** **(15)**

(wherein Y² is the same as Y² in the general formula (5); and c corresponds to the carbon atom to which R¹, R² and Y bind in the general formula (2)).
Similarly, when the active hydrogen-containing compound is the phosphinide group-containing compound, the carboxyl group-containing compound, the methylenebiscarbonyl group-containing compound, the nitro group-containing compound, the cyano group-containing compound, the methylenebiscyano group-containing compound or the azide group-containing compound, it is represented by the following general formula (16) to (22), respectively. Y³ to Y¹³ in the general formulae (16) to (21) are the same as Y³ to Y¹³ in the above general formulae (6) to (11). c corresponds to, as in the above (14) and (15), the carbon atom to which R¹, R² and Y bind in the general formula (2).

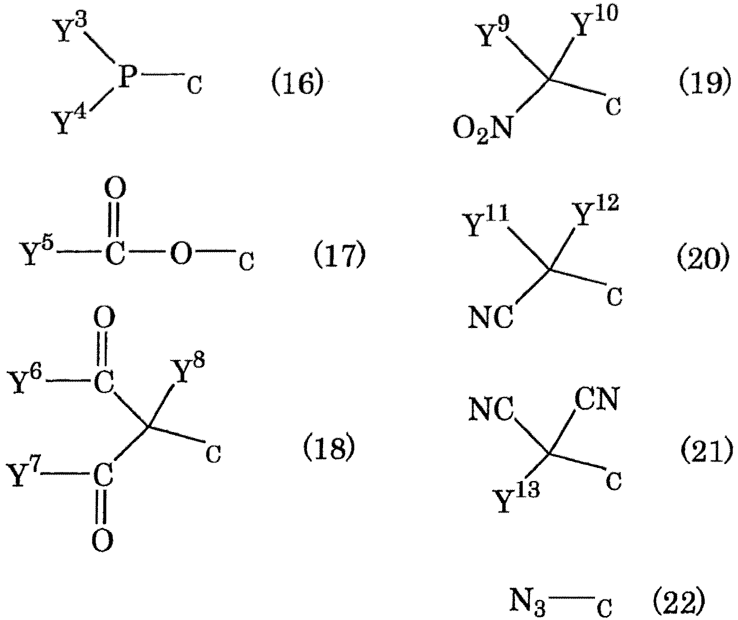

The reaction material of the present invention, the compound represented by the general formula (1), can be synthesized by, for example, the Morita-Baylis-Hillman reaction which is the addition reaction of acrylate esters to electrophilic compounds. More specifically, the compound of the general formula (1) wherein R¹ and R² are a hydrogen atom, R³ is a methyl group and X is a hydroxy group can be synthesized by addition reaction of methyl acrylate to formaldehyde.

The acrylate esters may include, for example, methyl acrylate, ethyl acrylate, n-propyl acrylate, i-propyl acrylate, n-butyl acrylate, n-amyl acrylate, s-amyl acrylate, t-amyl acrylate, neopentyl acrylate, n-hexyl acrylate, s-hexyl acrylate, n-heptyl acrylate, n-octyl acrylate, s-octal acrylate, t-octyl acrylate, 2-ethylhexyl acrylate, capryl acrylate, nonyl acrylate, decyl acrylate, undecyl acrylate, lauryl acrylate, tridecyl acrylate, myristyl acrylate, pentadecyl acrylate, cetyl acrylate, heptadecyl acrylate, stearyl acrylate, nonadecyl acrylate, eicosyl acrylate, seryl acrylate, melissyl acrylate, vinyl acrylate, allyl acrylate, metallyl acrylate, crotyl acrylate, 1,1-dimethyl-2-propenyl acrylate, 2-methylbutenyl acrylate, 3-methyl-2-butenyl acrylate, 3-methyl-3-butenyl acrylate, 2-methyl-3-butenyl acrylate, oleyl acrylate, linoleic acrylate, linolenic acrylate, cyclopropyl acrylate, cyclopentyl acrylate, cyclopentylmethyl acrylate, cyclohexyl acrylate, cyclohexylmethyl acrylate, 4-methylcyclohexyl acrylate, 4-t-butylcyclohexyl acrylate, tricyclodecanyl acrylate, isobornyl acrylate, adamantyl acrylate, dicyclopentanyl acrylate, dicyclopentenyl acrylate, phenyl acrylate, methylphenyl acrylate, dimethylphenyl acrylate, trimethylphenyl acrylate, 4-t-butylphenyl acrylate, benzyl acrylate, diphenylmethyl acrylate, diphenylethyl acrylate, triphenylmethyl acrylate, cinnamyl acrylate, naphthyl acrylate, anthranyl acrylate, methoxyethyl acrylate, methoxyethoxyethyl acrylate, methoxyethoxyethoxyethyl acrylate, 3-methoxybutyl acrylate, ethoxyethyl acrylate, ethoxyethoxyethyl acrylate, cyclopentoxyethyl acrylate, cyclohexyloxyethyl acrylate, cyclopentoxyethoxyethyl acrylate, cyclohexyloxyethoxyethyl acrylate, dicyclopentenyloxyethyl acrylate, phenoxyethyl acrylate, phenoxyethoxyethyl acrylate, glycidyl acrylate, β-methylglycidyl acrylate, β-ethylglycidyl acrylate, 3,4-epoxycyclohexylmethyl acrylate, 2-oxycetanmethyl acrylate, 3-methyl-3-oxycetanmethyl acrylate, 3-ethyl-3-oxycetanmethyl acrylate, tetrahydrofuranyl acrylate, tetrahydrofurfuryl acrylate, tetrahydropyranyl acrylate, dioxazolanyl acrylate, dioxanyl acrylate and the like. Among these, methyl acrylate, ethyl acrylate, n-propyl acrylate, i-propyl acrylate, n-butyl acrylate, n-amyl acrylate, s-amyl acrylate, t-amyl acrylate, neopentyl acrylate, n-hexyl acrylate, s-hexyl acrylate, n-heptyl acrylate, n-cetyl acrylate, s-octyl acrylate, t-octyl acrylate, 2-ethylhexyl acrylate, capryl acrylate, nonyl acrylate, decyl acrylate, undecyl acrylate, lauryl acrylate, tridecyl acrylate, myristyl acrylate, pentadecyl acrylate, cetyl acrylate, heptadecyl acrylate, stearyl acrylate, nonadecyl acrylate, eicosyl acrylate, seryl acrylate, melissyl acrylate, vinyl acrylate, allyl acrylate, metallyl acrylate and crotyl acrylate are preferred. One or two or more of these acrylate esters may be used.

The electrophilic compounds may include, for example, formaldehyde, paraformaldehyde, trioxane, acetaldehyde, propionaldehyde, butylaldehyde, trimethylacetaldehyde, acrolein, crotonaldehyde, cyclohexanealdehyde, furfural, furylacrolein, benzaldehyde, terephthalaldehyde, phenylacetaldehyde, α-phenylpropylaldehyde, β-phenylpropylaldehyde, o-hydroxybenzaldehyde, m-hydroxybenzaldehyde, p-hydroxybenzaldehyde, o-methylbenzaldehyde, m-methylbenzaldehyde, p-methylbenzaldehyde, o-chlorobenzaldehyde, m-chlorobenzaldehyde, p-chlorobenzaldehyde, cinnamic aldehyde and the like. Among these, formaldehyde, paraformaldehyde, trioxane, acetaldehyde, propionaldehyde, butylaldehyde and benzaldehyde are preferred. More preferred are formaldehyde, paraformaldehyde, trioxane and benzaldehyde and still more preferred are formaldehyde, paraformaldehyde and trioxane. One or two or more of these electrophilic compounds may be used.

When the compound represented by the general formula (1) is synthesized by the Morita-Baylis-Hillman reaction, the mixing ratio of the acrylate ester and the electrophilic compound is preferably, relative to 100% by mass of the acrylate ester, 1000 to 10% by mass of the electrophilic compound. Due to such a mixing ratio, the compound represented by the general formula (1) can be appropriately synthesized. It is more preferably 500 to 20% by mass and still more preferably 200 to 50% by mass.

In the synthesis reaction of the compound represented by the general formula (1), a catalyst may be used which is not particularly limited as long as it can be used for the Morita-Baylis-Hillman reaction described in the following reference and may include, for example, nitrogen-containing compounds such as the above tertiary amines and the like; phosphorous compounds such as trimethyl phosphine, triethyl phosphine, tributyl phosphine and the like; oxygen compounds; sulfur compounds; selenic compounds; fluorine compounds; chlorine compounds; bromine compounds; iodine compounds and the like.
Among these, it is preferable to use a tertiary amine such as trimethylamine, triethylenediamine, quinuclidine, pyrocollidine or the like because the α-substituted acrylate ester represented by the general formula (2) can be synthesized in one step by charging together the active hydrogen-containing compound and the acid and/or the salt thereof used in the present invention, or alternatively, the α-substituted acrylate ester represented by the general formula (2) can be synthesized by adding, after the synthesis reaction of the compound represented by the general formula (1), the active hydrogen-containing compound and the acid and/or the salt thereof used in the present invention without purification step.
Deevi Basavaiah and two others., "Chemical Review", 2003, Vol. 103, p.811-891

The amount of the catalyst in the synthesis reaction of the compound represented by the general formula (1) is preferably, relative to 100% by mass of the acrylate ester, 50 to 0.1% by mass. Such a mixing ratio allows appropriate synthesis of the compound represented by the general formula (1). It is more preferably 20 to 0.5% by mass and still more preferably 10 to 1% by mass.

The synthesis reaction of the compound represented by the general formula (1) may be carried out in a solvent. The solvent used may be the same as the solvent described above for synthesis of the α-substituted acrylate ester of the present invention. Among these, dipropyl ether, diisopropyl ether, dibutyl ether, tetrahydrofuran, tetrahydropyran, dioxane, toluene, xylene, cyclohexane, hexane, methylcyclohexane, heptane, octane and water are preferred. One or two or more of these solvents may be used.

The amount of the solvent to be used in the synthesis reaction of the compound represented by the general formula (1) is preferably, relative to 100% by mass of the acrylate ester, 10000 to 0% by mass. Such a mixing ratio allows appropriate synthesis of the compound represented by the general formula (1). It is more preferably 1000 to 0% by mass and still more preferably 100 to 0% by mass.

The present invention is also directed to the α-substituted acrylate ester obtained the above method, which is a product obtained by a reaction in which the elimination of the Lewis base functional group in the compound represented by the general formula (1) and replacement by a residue of the active hydrogen-containing compound undergo predominantly over transesterification at the position of -COOR³.
The term " obtained...undergo predominantly " refers to the fact that in a product mixture obtained after the reaction, the amount of the compound (target compound) obtained by the reaction in which the Lewis base functional group in the compound represented by the general formula (1) is eliminated and replaced by a residue of the active hydrogen-containing compound is higher than that of the compound (byproduct) obtained by transesterification reaction at the position of -COOR³ of the compound represented by the general formula (1). Because the α-substituted acrylate ester of the present invention is obtained by the above method of the present invention, it has less proportion of the compound obtained by the transesterification reaction at the position of -COOR³ of the compound represented by the general formula (1) compared to the α-substituted acrylate esters obtained by the conventional production methods and therefore can be said that it can be more suitably applied to various applications described above.

The ratio, in the product mixture obtained after the reaction, between the target product and the compound (byproduct) obtained by transesterification reaction at the position of -COOR³ of the compound represented by the general formula (1) is preferably 100/0 to 3/2. If the ratio is in this range, the product has sufficiently high selectivity and purification procedures such as distillation may be facilitated, which is industrially preferable. It is more preferably 100/0 to 2/1 and still more preferably 100/0 to 5/1.

When R³ in the general formula (1) is an organic group having 1 to 200 carbon atoms and the active hydrogen-containing compound is alcoholic hydroxy group-containing compound and/or phenolic hydroxy group-containing compound, the α-substituted acrylate esters obtained according to the method of the present invention and by the conventionally known methods contain byproduct in which the moiety other than the Lewis base functional group in the compound represented by the general formula (1) binds to the organic group represented by R³ via an ether bond, namely, the compound (the compound represented by the general formula (23) described hereinbelow) in which the Lewis base functional group (X) in the compound represented by the general formula (1) is replaced by -OR³. However, the α-substituted acrylate ester obtained according to the method of the present invention contains less amount of the above byproduct than the α-substituted acrylate esters obtained according to the conventionally known methods.

The amount of the compound (byproduct) represented by the general formula (23) which is contained in the α-substituted acrylate ester is preferably, relative to 100% by mass of the α-substituted acrylate ester, 0.001% by mass to 0.5% by mass, more preferably 0.01% by mass to 0.45% by mass and the most preferably 0.05% by mass to 0.4% by mass.
Accordingly, the present invention is also directed to a composition comprising an α-substituted acrylate ester represented by the following general formula (2');

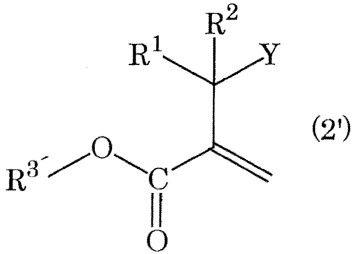

(wherein R¹ and R² are the same or different and represent a hydrogen atom or an organic group having 1 to 200 carbon atoms; R³' represents an organic group having 1 to 200 carbon atoms; and Y represents a residue of the active hydrogen-containing compound), wherein the composition comprises a compound represented by the following general formula (23);

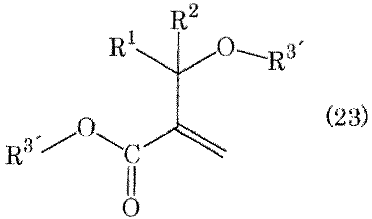

(wherein R¹, R² and R³' are the same as those in the above general formula (2')), and the composition comprises the compounds represented by the general formula (23) at 0.001 to 0.5% by mass relative to 100% by mass of the α-substituted acrylate ester represented by the general formula (2').

The composition comprising the α-substituted acrylate ester preferably comprises the compound represented by the following general formula (1');

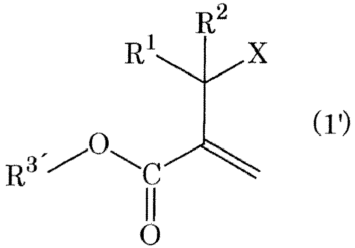

(wherein R¹ and R² are the same or different and represent a hydrogen atom or an organic group having 1 to 200 carbon atoms; R³' represents an organic group having 1 to 200 carbon atoms; and X represents the Lewis base functional group), at 10 to 0.01% by mass relative to 100% by mass of the α-substituted acrylate ester represented by the general formula (2'). When the amount of the unreacted material, which is the compound represented by the general formula (1'), in the composition after production of the α-substituted acrylate ester is in the above range, it can be said that the amount of the unreacted material after production is low. It is known that the unreacted material is difficult to be removed. Therefore, it is advantageous because the α-substituted acrylate ester having high purity can be obtained. The amount of the compound represented by the general formula (1') in the composition is preferably 5 to 0.01% by mass and still more preferably 2 to 0.01% by mass.

### - Effects of the Invention

The method for producing the α-substituted acrylate ester of the present invention has the above configurations and allows the reaction in a shorter time with high yield, thus it can be suitably used as a method for industrially producing α-substituted acrylate esters. More specifically, the double bond moiety of the compound represented by the general formula (1) can be activated and can easily react with the active hydrogen-containing compound when the acid and/or the salt thereof of the present invention coexists during the reaction in which the Lewis base functional group in the compound represented by the general formula (1) is eliminated and replaced by a residue of the active hydrogen-containing compound. In addition, because the reaction goes through a intermediate which is stable and has low basicity, production of the intermediate is promoted as well as side reactions which are promoted by bases such as transesterification reaction and hydrolysis reaction are reduced. Further, inactivation reaction of the tertiary amine catalyst is suppressed and therefore the above reaction proceeds in a short time without addition of a high amount of the catalyst. As a result of these, less reactive and inexpensive substrates can be used as starting materials, high yield and selectivity can be obtained in a short time and therefore the industrially suitable method for producing can be provided without the problems in material cost, apparatus corrosion or necessity of waste detoxification which are caused by using highly active materials such as halomethyl acrylate esters. In addition, these effects result from improvements in the catalyst cycle which is common for reactions where the Lewis base functional group in the compound represented by the general formula (1) is eliminated and replaced by a residue of the active hydrogen-containing compound. Therefore, it is believed that the same effects are obtained as far as the above reactions are carried out.

### (BRIEF DESCRIPTION OF the DRAWINGS)

Fig. 1 is a schematic view of the reaction mechanism which may proceed in the method for producing an α-substituted acrylate ester of the present invention;
Fig. 2 is a schematic view of some examples of reaction pathways between synthesis of the compound represented by the general formula (1) and synthesis of the α-substituted acrylate ester represented by the general formula (2); and
Fig. 3 is a schematic view of the outline of the reaction apparatus used in Example 28.

### DESCRIPTION OF EMBODIMENTS

The present invention is further described in detail by giving Examples hereinbelow, however, the present invention is not limited only to the examples. Unless otherwise specified, "part(s)" and "%" respectively mean "part(s) by weight" and "% by mass".

In the following Examples and Comparative Examples, material conversion rate, yield and selectivity were measured and determined as follows.
The material conversion rate and yield were measured by using a gas chromatograph (product name: "AT-6850", Agilent Technologies, capillary column: DB-WAX (trade name, Agilent Technologies, length: 30 m, inner diameter: 0.25 mm, film thickness: 0.25 µm)) and quantified by using a preliminarily prepared calibration curve.
The selectivity was determined according to the following equation.
Selectivity = Yield (mol%)/Material conversion rate (mol%)

### (Comparative Example 1)

To a tube with screw cap (20 ml), 2.3 g of methyl α-hydroxymethylacrylate, 1.7 g (1.5 equivalents) of allylalcohol, 0.2 g (0.1 equivalents) of triethylenediamine (DABCO) as the catalyst tertiary amine, 0.001 g of hydroquinone monomethyl ether as the polymerization inhibitor and 0.001 g of 4-hydroxy-2,2,6,6-tetramethylpiperidine-1-oxyl were added. The tube was sealed before raising the temperature of the reaction solution to 80°C and the reaction was carried out for 16 hours.
The reaction solution was analyzed after 4, 8 and 16 hours according to the above methods to determine the conversion rate of methyl α-hydroxymethylacrylate and the yield and reaction selectivity of methyl α-allyloxymethylacrylate relative to methyl α-hydroxymethylacrylate. In addition, the yield and reaction selectivity of the byproduct, the transesterification product of methyl α-hydroxymethylacrylate, relative to methyl α-hydroxymethylacrylate were also determined. The results are shown in Table 1.

### (Examples 1 to 25)

The reactions and analyses were carried out in the same manner as Comparative Example 1 except that the catalysts shown in Table 1 were used. The results are shown in Table 1. In Table 1, values of the amount used of the tertiary amine, the amount used of the acid, the material conversion rate and the yield are represented as molar ratio relative to 100 ml% of methyl α-hydroxymethylacrylate. With regard to the tertiary amine and acid, the values in brackets represent the amounts used. Abbreviations in Table 1 represent the followings.
DABCO: Triethylenediamine
TMA: Trimethylamine
AcOH: Acetic acid
PhCO₂H: Benzoic acid
(CO₂H)₂: Oxalic acid
TCP: 2,4,6-Trichlorophenol
MQ: Paramethoxyphenol
TolSO₂H: p-Toluenesulfinic acid
B(OH)_{3:} Boric acid
Zn(OAc)₂: Zinc acetate
Zn(acac)₂: Zinc acetylacetonate
Zn(OTf)₂: Zinc triflate
Mg(OAc)₂: Magnesium acetate
La(OAc)₃: Lanthanum acetate
Al(OTf)₃: Aluminum triflate
TMAHCl: Trimethylamine hydrochloride
HClaq., TEA: Mixture of hydrochloric acid and triethylamine, AcOH, TEA: Mixture of acetic acid and triethylamine B(OH)₃, TEA: Mixture of boric acid and triethylamine TsOH, TEA: Mixture of p-toluenesulfonic acid and triethylamine H₃PO₄, TEA: Mixture of phosphoric acid and triethylamine H₂SO₄, TEA: Mixture of sulfuric acid and triethylamine B(OH)₃, Pyridine: Mixture of boric acid and pyridine
H₃PO₄: Phosphoric acid
AMA: Methyl α-allyloxymethylacrylate
Transesterification product: Transesterification product of methyl α-hydroxymethylacrylate

**[Table 1]**

| | Catalyst | | | | Material conversion (mol%) | | | Yield of AMA (mol%) | | | Selectivity of AMA | | | Yield of transesterification product (mol%) | | | Selectivity of transesterification product | | |
|---|---|---|---|---|---|---|---|---|---|---|---|---|---|---|---|---|---|---|---|
| | Tertiary amine | Acid (mol%) | | pKa | 4h | 8h | 16h | 4h | 8h | 16h | 4h | 8h | 16h | 4h | 8h | 16h | 4h | 8h | 16h |
| Comparative Example 1 | DABCO (10mol%) | - | | | 29 | 48 | 69 | 10 | 21 | 37 | 0.35 | 0.43 | 0.53 | 2.1651 | 3.2589 | 4.393 | 0.08 | 0.07 | 0.06 |
| Example 1 | | | AcOH(15) | 4.8 | 63 | 79 | 88 | 39 | 59 | 70 | 0.62 | 0.75 | 0.80 | 0.3825 | 0.6162 | 0.731 | 0.01 | 0.01 | 0.01 |
| Example 2 | | | PhCO₂H(10) | 4.2 | 63 | 78 | 92 | 37 | 57 | 69 | 0.59 | 0.73 | 0.75 | 0.4416 | 0.6684 | 0.9292 | 0.01 | 0.01 | 0.01 |
| Example 3 | | | (CO₂H)₂(10) | 1.3,4.3 | 31 | 49 | 73 | 14 | 24 | 40 | 0.45 | 0.49 | 0.54 | 0 | 0.0438 | 0.0928 | 0.00 | 0.00 | 0.00 |
| Example 4 | | Broensted acid | TCP(10) | 6.2 | 60 | 76 | 87 | 34 | 54 | 68 | 0.57 | 0.71 | 0.78 | 0.2527 | 0.9946 | 1.435 | 0.00 | 0.01 | 0.02 |
| Example 5 | | | MQ(10) | 10.2 | 33 | 55 | 74 | 13 | 24 | 40 | 0.40 | 0.43 | 0.54 | 1.7945 | 2.5309 | 3.2238 | 0.05 | 0.05 | 0.04 |
| Example 6 | | | TolSO₂H(10) | (7) | 23 | 41 | 63 | 20 | 32 | 46 | 0.85 | 0.79 | 0.73 | 1.0901 | 1.5663 | 2.1083 | 0.05 | 0.04 | 0.03 |
| Example 7 | | | B(OH)₃(10) | 9.2 | 74 | 83 | 89 | 45 | 58 | 69 | 0.60 | 0.70 | 0.77 | 1.3211 | 2.1028 | 2.9667 | 0.02 | 0.03 | 0.03 |
| Example 8 | | | Zn(OAc)₂(10) | | 91 | 93 | 95 | 50 | 46 | 54 | 0.55 | 0.50 | 0.57 | 5.3379 | 6.137 | 7.8082 | 0.06 | 0.07 | 0.08 |
| Example 9 | | | Zn(OAc)₂(2) | | 87 | 91 | 93 | 60 | 67 | 65 | 0.69 | 0.74 | 0.70 | 2.4618 | 3.5691 | 4.9961 | 0.03 | 0.04 | 0.05 |
| Example 10 | | | Zn(acac)₂(2) | | 88 | 93 | 95 | 43 | 49 | 49 | 0.49 | 0.53 | 0.52 | 5.6804 | 7.2722 | 8.7941 | 0.06 | 0.09 | 0.09 |
| Example 11 | DABCO (10mol%) | Lewis acid | Zn(OTf)₂(2) | | 63 | 82 | 91 | 33 | 55 | 65 | 0.52 | 0.67 | 0.71 | 1.6082 | 2.7762 | 4.5455 | 0.03 | 0.03 | 0.05 |
| Example 12 | | | Mg(OAc)₂(2) | | 38 | 60 | 77 | 15 | 28 | 43 | 0.39 | 0.47 | 0.56 | 2.0139 | 3.2195 | 4.4485 | 0.05 | 0.05 | 0.06 |
| Example 13 | | | La(OAc)₃(2) | | 47 | 68 | 82 | 18 | 32 | 46 | 0.39 | 0.47 | 0.56 | 3.4687 | 4.649 | 5.6411 | 0.07 | 0.07 | 0.07 |
| Example 14 | | | Al(OTf)₃(2) | | 57 | 69 | 78 | 27 | 38 | 49 | 0.48 | 0.55 | 0.63 | 1.7435 | 2.0833 | 2.4556 | 0.03 | 0.03 | 0.03 |
| Example 15 | | | TMAHCl(10) | | 40 | 61 | 77 | 31 | 46 | 55 | 0.77 | 0.76 | 0.72 | 1.424 | 1.9904 | 2.455 | 0.04 | 0.03 | 0.03 |
| Example 16 | | | HCl aq.(10), TEA(10) | | 50 | 67 | 77 | 23 | 35 | 46 | 0.46 | 0.52 | 0.60 | 1.6947 | 2.426 | 3.9321 | 0.03 | 0.04 | 0.05 |
| Example 17 | | | AcOH(10), TEA(10) | | 54 | 72 | 85 | 28 | 44 | 58 | 0.52 | 0.62 | 0.68 | 1.5014 | 2.2326 | 3.1113 | 0.03 | 0.03 | 0.04 |
| Example 18 | | Broensted acid + Amines | B(OH)3(10), TEA(10) | | 69 | 80 | 88 | 38 | 50 | 58 | 0.55 | 0.62 | 0.66 | 2.3031 | 3.747 | 5.2935 | 0.03 | 0,05 | 0.06 |
| Example 19 | | | TsOH(10), TEA(10) | | 53 | 67 | 80 | 25 | 38 | 49 | 0.47 | 0.57 | 0.62 | 1.7293 | 2.4934 | 3.1219 | 0.03 | 0.04 | 0.04 |
| Example 20 | | | H3PO4(5), TEA(10) | | 58 | 70 | 82 | 27 | 37 | 51 | 0.47 | 0.53 | 0.62 | 2.7138 | 3.9338 | 5.4512 | 0.05 | 0.06 | 0.07 |
| Example 21 | | | H2SO4(15), TEA(10) | | 41 | 61 | 76 | 18 | 28 | 43 | 0.44 | 0.46 | 0.56 | 2.2207 | 3.2314 | 4.2653 | 0,05 | 0.05 | 0.06 |
| Example 22 | | | B(OH)3(10), Pyridine(10) | | 70 | 78 | 86 | 40 | 53 | 65 | 0.56 | 0.68 | 0.76 | 1.2005 | 1.8432 | 2.5894 | 0.02 | 0.02 | 0.03 |
| Example 23 | | | B(OH)₃(10) | 9.2 | 78 | 85 | - | 42 | 53 | - | 0.54 | 0.62 | | 6.3677 | 8.5339 | - | 0.08 | 0.10 | - |
| Example 24 | TMA (30mol%) | Broensted acid | AcOH(10) | 4.8 | 57 | 71 | 83 | 25 | 36 | 43 | 0.43 | 0.51 | 0.52 | 2.797 | 3.7797 | 3.0625 | 0.05 | 0.05 | 0.04 |
| Example 25 | | | H₃PO₄(10) | 2.2,7.2 | - | - | 87 | - | - | 47 | - | - | 0.54 | - | - | 4.9213 | - | | 0.06 |

### (Example 26)

To a round-bottom flask (500 ml), 203.2 g of methyl α-hydroxymethylacrylate, 9.8 g (0.1 equivalents) of triethylenediamine (DABCO) as the catalyst tertiary amine, 10.9 g of boric acid as the acid, 0.1 g of hydroquinone monomethyl ether as the polymerization inhibitor and 0.1 g of 4-hydroxy-2,2,6,6-tetramethylpiperidine-1-oxyl were added. The reaction solution was heated to 100°C while blowing nitrogen containing 7% oxygen at 5 ml per minute. After reduction of pressure to 10 kPa, the reaction was carried out for 2 hours while distilling produced water off. After recovering normal pressure, analyses of the reaction solution according to the above methods revealed that the conversion rate of methyl α-hydroxymethylacrylate was 89 mol% and the yield of the ether dimer relative to methyl α-hydroxymethylacrylate was 81 mol%. Then, a mixed solution of 9.8 g of triethylenediamine (DABCO) and 152.5 g (1.5 equivalents) of allylalcohol was fallen in drop to the reaction solution at 100°C for 2 hours, which was then aged at 100°C for 12 hours. Analyses of the reaction solution after aging according to the above methods revealed that the conversion rate of methyl α-hydroxymethylacrylate was 92 mol% and the yield of methyl α-allyloxymethylacrylate relative to methyl α-hydroxymethylacrylate was 65 mol%. The thus obtained reaction solution was washed with water and distilled to give methyl α-allyloxymethylacrylate containing 0.2 wt% of methyl α-methoxymethylacrylate.

### (Comparative Example 2)

To a round-bottom flask (500 ml), 203.2 g of methyl α-hydroxymethylacrylate, 152.5 g of allylalcohol, 19.6 g (0.1 equivalents) of triethylenediamine (DABCO) as the catalyst tertiary amine, 0.1 g of hydroquinone monomethyl ether as the polymerization inhibitor and 0.1 g of 4-hydroxy-2,2,6,6-tetramethylpiperidine-1-oxyl were added. The reaction solution was heated to 100°C while blowing nitrogen containing 7% oxygen at 5 ml per minute. The conversion rate of methyl α-hydroxymethylacrylate, the yield of methyl α-allyloxymethylacrylate relative to methyl α-hydroxymethylacrylate, the selectivity of methyl α-allyloxymethylacrylate, the yield of methyl α-methoxymethylacrylate and the residual rate of DABCO are shown in Table 2. The thus obtained reaction solution was washed with water and distilled to give methyl α-allyloxymethylacrylate containing 0.6 wit% of methyl α-methoxymethylacrylate. A polymer component derived from methyl α-methoxymethylacrylate was produced in the distillation column, so that further distillation operation was difficult.

### (Example 27)

To a round-bottom flask (500 ml), 203.2 g of methyl α-hydroxymethylacrylate, 152.5 g of allylalcohol, 9.8 g (0.05 equivalents) of triethylenediamine (DABCO) as the catalyst tertiary amine, 10. 6 g (0.1 equivalents) of acetic acid as the Broensted acid, 0.1. g of hydroquinone monomethyl ether as the polymerization inhibitor and 0.1 g of 4-hydroxy-2,2,6,6-tetramethylpiperidine-1-oxyl were added. The reaction solution was heated to 100°C while blowing nitrogen containing 7% oxygen at 5 ml per minute. The conversion rate of methyl α-hydroxymethylacrylate, the yield of methyl α-allyloxymethylacrylate relative to methyl α-hydroxymethylacrylate, the selectivity of methyl α-allyloxymethylacrylate, the yield of methyl α-methoxymethylacrylate and the residual rate of DABCO are shown in Table 2. The thus obtained reaction solution was washed with water and distilled to give methyl α-allyloxymethylacrylate containing 0.05 wt% of methyl α-methoxymethylacrylate.

### (Example 28)

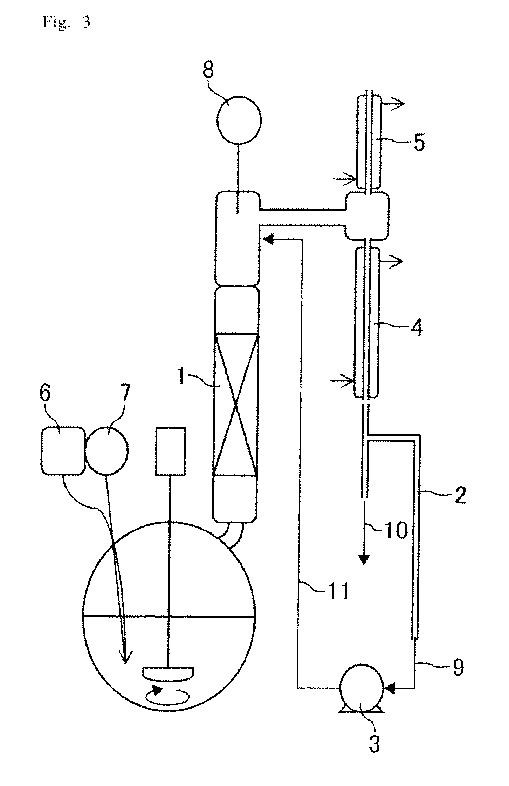

To a round-bottom flask (capacity: 5 L) equipped with a distillation column 1, a phase separator 2, a reflux pump 3 and the like as shown in Fig. 3, 1625.7 g of methyl α-hydroxymethylacrylate, 1219.7 g (1.5 equivalents) of allylalcohol, 78.5 g (0.05 equivalents) of triethylenediamine (DABCO) as the catalyst tertiary amine, 84. 9 g (0.1 equivalents) of acetic acid as the Broensted acid, 30.7 g (0.01 equivalents) of zinc acetate dihydrate as the Lewis acid, 429.1 g (0.3 equivalents) of diisopropyl ether as the azeotropic agent of water, 0.8 g of hydroquinone monomethyl ether as the polymerization inhibitor and 0.8 g of 4-hydroxy-2,2,6,6-tetramethylpiperidine-1-oxyl were added. The reaction solution was heated to 90°C at normal pressure while blowing nitrogen gas 6 containing 7% oxygen at 5 ml per minute. The column top liquid was separated into an oil phase 9 and an aqueous phase 10 in the phase separator 2 and the oil phase 9 was returned to the column top as reflux solution 11 while the aqueous phase 10 was removed. The conversion rate of methyl α-hydroxymethylacrylate, the yield of methyl α-allyloxymethylacrylate relative to methyl α-hydroxymethylacrylate, the selectivity of methyl α-allyloxymethylacrylate, the yield of methyl α-methoxymethylacrylate and the residual rate of DABCO are shown in Table 2. The thus obtained reaction solution was washed with water and distilled to give methyl α-allyloxymethylacrylate containing 0.02 wt% of methyl α-methoxymethylacrylate.
Abbreviations in Table 2 represent the followings.
AMA: Methyl α-allyloxymethylacrylate
Methyl ether: Methyl α-methoxymethylacrylate
DABCO: Triethylenediamine

**[Table 2]**

| (unit: mol%) | | | | | | | |
|---|---|---|---|---|---|---|---|
| Reaction time (time) | | 6 | 8 | 10 | 12 | 14 | 16 |
| Comparative Example 2 | Material conversion | 64.6 | 73.7 | 80.1 | 83.9 | 84.9 | - |
| | Yield of AMA | 31.3 | 39.1 | 44.2 | 46.8 | 49.0 | - |
| | Selectivity of AMA | 48.5 | 53.1 | 55.2 | 55.8 | 57.7 | - |
| | Yield of methyl ether | 2.1 | 3.0 | 3.6 | 3.9 | 4.1 | - |
| | Residual rate of DABCO | 52.1 | 54.4 | 48.6 | 44.5 | 42.9 | - |
| Example 27 | Material conversion | 71.8 | 78.2 | 81.5 | 83.9 | 85.6 | - |
| | Yield of AMA | 48.1 | 56.2 | 60.5 | 63.4 | 65.0 | - |
| | Selectivity of AMA | 66.9 | 71.9 | 74.3 | 75.5 | 75.8 | - |
| | Yield of methyl ether | 0.6 | 0.8 | 0.9 | 1.0 | 1.1 | - |
| | Residual rate of DABCO | 79.6 | 75.9 | 71.9 | 69.0 | 66.4 | - |
| Example 28 | Material conversion | 83.5 | 89.1 | 93.1 | 95.4 | 96.9 | 97.9 |
| | Yield of AMA | 60.8 | 70.4 | 77.9 | 81.7 | 85.2 | 87.0 |
| | Selectivity of AMA | 72.8 | 79.0 | 83.7 | 85.6 | 87.9 | 88.9 |
| | Yield of methyl ether | 0.6 | 0.7 | 0.8 | 0.8 | 0.7 | 0.7 |
| | Residual rate of DABCO | 92.0 | 93.2 | 83.8 | 91.5 | 81.2 | 82.4 |

Based on the results of the above Examples and Comparative Examples, it was found that when the compound represented by the general formula (1) and the active hydrogen-containing compound are reacted to produce the α-substituted acrylate ester represented by the general formula (2) the reaction carries out under the condition such that the tertiary amine and the acid and/or the salt thereof coexist, the α-substituted acrylate ester can be obtained in a short reaction time with high yield and selectivity compared to the reaction carried out in the presence of the tertiary amine.
Among Examples, in Examples 8, 10 and 23 in which the α-substituted acrylate ester was obtained in a particularly short time with high yield, the reaction for a prolonged time resulted in a higher ratio of the transesterification product of methyl α-hydroxymethylacrylate, i.e., byproduct, relative to methyl α-allyloxymethylacrylate than that in Comparative Example 1 which was carried out in the presence of the tertiary amine. This indicates that, in the method of the present invention in which methyl α-allyloxymethylacrylate is produced preferentially in a particularly short time, further continuation of the reaction may increase the ratio of byproducts.
In the above Examples, the specific compound represented by the general formula (1) and the specific active hydrogen-containing compound were used. However, the reaction mechanism which is believed to proceed when the compound represented by the general formula (1) reacts with the active hydrogen-containing compound in the coexistence of the tertiary amine and the acid and/or a salt thereof is as shown in Fig. 1. Therefore, when the reaction is carried out under the condition where the tertiary amine and the acid and/or the salt thereof coexist, all reactions proceed in the same reaction mechanism.
Accordingly, it can be concluded that the present invention can be applied to throughout the technical scope of the invention and to various embodiments disclosed herein and exhibit advantageous effects based on the results of the above Examples and Comparative Examples.

### (REFERENCE LIST)

- 1:: Distillation column
- 2:: Phase separator
- 3:: Reflux pump
- 4, 5:: Condenser
- 6:: Gas
- 7, 8:: Thermometer
- 9:: Oil phase
- 10:: Aqueous phase
- 11:: Reflux solution

## Claims

1. A method for producing an α-substituted acrylate ester represented by the following general formula (2) : 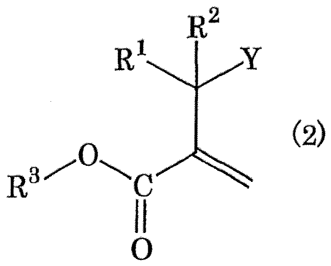 (wherein R¹, R² and R³ are the same or different and represent a hydrogen atom or an organic group having 1 to 200 carbon atoms; and Y represents a residue of an active hydrogen-containing compound) obtained by reacting a compound represented by the following general formula (1); 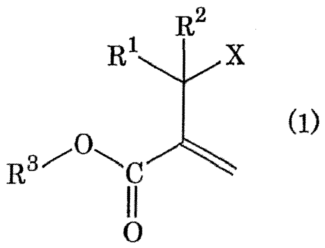 (wherein R¹, R² and R³ are the same as those in the above general formula (2); and X represents a Lewis base functional group) with the active hydrogen-containing compound,
wherein the method comprises a step of carrying out the reaction under a condition where a tertiary amine and an acid and/or a salt thereof coexist, and
essentially comprises a reaction in which the Lewis base functional group in the compound represented by the general formula (1) is eliminated and replaced by the residue of the active hydrogen-containing compound.

2. The method for producing an α-substituted acrylate ester according to claim 1,
wherein in the step of carrying out the reaction, the molar ratio of the tertiary amine and the acid and/or the salt thereof is 10000/1 to 1/10000.

3. The method for producing an α-substituted acrylate ester according to claim 1 or 2,
wherein the active hydrogen-containing compound is at least one compound selected from the group consisting of alcoholic hydroxy group-containing compounds, phenolic hydroxy group-containing compounds, mercapto group-containing compounds, phosphinide group-containing compounds, carboxyl group-containing compounds, methylenebiscarbonyl group-containing compounds, nitro group-containing compounds, cyano group-containing compounds, methylenebiscyano group-containing compounds and azide group-containing compounds.

4. The method for producing an α-substituted acrylate ester according to any of claims 1 to 3,
wherein, when the active hydrogen-containing compound is the alcoholic hydroxy group-containing compound and/or the phenolic hydroxy group-containing compound represented by Y¹-OH (where Y¹ represents an organic group having 1 to 200 carbon atoms), Y¹ is different from R³.

5. An α-substituted acrylate ester obtained by the method according to any of claims 1 to 4, which is a product obtained by a reaction in which the elimination of the Lewis base functional group in the compound represented by the general formula (1) and replacement by a residue of the active hydrogen-containing compounds undergo predominantly over transesterification at the position of -COOR³.

6. A composition containing an α-substituted acrylate ester represented by the following general formula (2'); 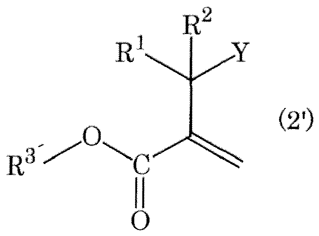 (wherein R¹ and R² are the same or different and represent a hydrogen atom or an organic group having 1 to 200 carbon atoms; R³' represents an organic group having 1 to 200 carbon atoms; and Y represents a residue of an active hydrogen-containing compound),
wherein the composition comprises a compound represented by the following general formula (23); 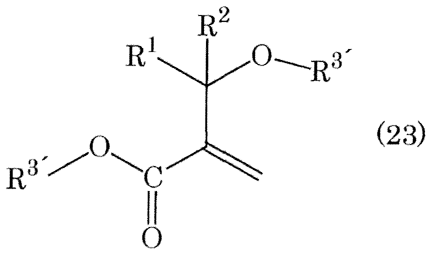 (wherein R¹, R² and R³' are the same as those in the above general formula (2')),
and the composition comprises the compound represented by the general formula (23) at 0.001 to 0.5% by mass relative to 100% by mass of the α-substituted acrylate ester represented by the general formula (2').

7. The composition containing an α-substituted acrylate ester according to claim 6,
wherein the composition comprises a compound represented by the following general formula (1'); 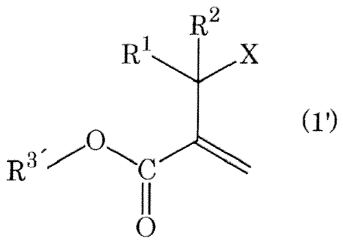 (wherein R¹ and R² are the same or different and represent a hydrogen atom or an organic group having 1 to 200 carbon atoms; R³' represents an organic group having 1 to 200 carbon atoms; and X represents a Lewis base functional group) at 10 to 0.01% by mass relative to 100% by mass of the α-substituted acrylate ester represented by the general formula (2').
